# FASCICULE DE BREVET EUROPEEN

(11) **EP 3 630 737 B1**
(45) Date de publication et mention de la délivrance du brevet: **01.09.2021**
(21) Numéro de dépôt: 18728347.8
(22) Date de dépôt: 28.05.2018
(51) Int. Cl.: C07D 265/16, C07F 5/02, C08G 73/06, C08G 75/0236, C08G 75/12

(54) **BENZOXAZINE BORÉE UTILISABLE POUR LA SYNTHÈSE DE POLYBENZOXAZINE**
BORIERTE BENZOXAZIN GEEIGNET FÜR DIE SYNTHESE VON POLYBENZOXAZIN
BORATED BENZOXAZINE USEFUL FOR THE SYNTHESIS OF POLYBENZOXAZINE

(30) Priorité: 31.05.2017 FR 1754810
(43) Date de publication de la demande: 08.04.2020
(73) Titulaire: COMPAGNIE GENERALE DES ETABLISSEMENTS MICHELIN, 63000 Clermont-Ferrand (FR)
(72) Inventeur: FEDURCO, Milan, 63040 Clermont-Ferrand cedex 9 (FR); RIBEZZO, Marco, 63040 Clermont-Ferrand cedex 9 (FR)
(74) Mandataire: Bocchi, Brigitte
(86) Numéro de dépôt international: PCT/EP2018/063965
(87) Numéro de publication internationale: WO 2018/219882

(56) Documents cités:
- WO-A1-2007/064801
- WO-A2-2010/002872
- JP-A- 2004 083 623

## Description

### 1. DOMAINE DE L'INVENTION

La présente invention est relative aux monomères utilisables pour la synthèse de résines thermodurcissables, destinées notamment à des systèmes adhésifs permettant en particulier le collage de métal à du caoutchouc.

Elle est plus particulièrement relative aux composés benzoxazine aptes à la synthèse de polybenzoxazines utilisables notamment comme couches adhésives dans des composites métal / caoutchouc destinés à la fabrication d'articles en caoutchouc tels que des bandages, pneumatiques ou non pneumatiques, pour véhicules.

### 2. ETAT DE LA TECHNIQUE

Les composites métal / caoutchouc, en particulier pour bandages pour véhicules, sont bien connus. Ils sont le plus souvent constitués d'une matrice en caoutchouc généralement diénique, réticulable au soufre, comportant des éléments de renforcement (ou « renforts ») métalliques tels que des fils, films ou câbles en acier au carbone.

Soumis à des contraintes très importantes lors du roulage des bandages, notamment à des compressions, flexions ou variations de courbure répétées, ces composites doivent de manière connue satisfaire à un grand nombre de critères techniques, parfois contradictoires, tels qu'uniformité, flexibilité, endurance en flexion et en compression, résistance à la traction, à l'usure et à la corrosion, et maintenir ces performances à un niveau très élevé aussi longtemps que possible.

On comprend aisément que l'interphase adhésive entre caoutchouc et renforts joue un rôle prépondérant dans la pérennité de ces performances. Le procédé traditionnel pour relier les compositions de caoutchouc à de l'acier au carbone consiste à revêtir la surface de l'acier avec du laiton (alliage cuivre-zinc), la liaison entre l'acier et la matrice de caoutchouc étant assurée par sulfuration du laiton lors de la vulcanisation ou cuisson du caoutchouc. Pour améliorer l'adhésion, on utilise en outre généralement, dans ces compositions de caoutchouc, des sels organiques ou des complexes de métal tels que des sels de cobalt, en tant qu'additifs promoteurs d'adhésion.

Or, on sait que l'adhésion entre l'acier au carbone et la matrice de caoutchouc est susceptible de s'affaiblir au cours du temps, du fait de l'évolution progressive des sulfures formés sous l'effet des différentes sollicitations rencontrées, notamment mécaniques et/ou thermiques, le processus de dégradation ci-dessus pouvant être accéléré en présence d'humidité. D'autre part, l'utilisation de sels de cobalt rend les compositions de caoutchouc plus sensibles à l'oxydation et au vieillissement, et en augmente significativement le coût, sans compter qu'il est souhaitable de supprimer à terme l'emploi de tels sels de cobalt dans les compositions de caoutchouc, en raison de l'évolution récente de la réglementation européenne sur ce type de sels métalliques.

Pour toutes les raisons exposées ci-dessus, les fabricants de composites métal / caoutchouc, en particulier les manufacturiers de bandages pour véhicules, sont à la recherche de solutions adhésives nouvelles pour faire coller les renforts métalliques aux compositions de caoutchouc, tout en palliant, au moins en partie, les inconvénients précités.

C'est ainsi que les demandes WO 2014/063963, WO 2014/063968, WO 2014/173838, WO 2014/173839 récemment publiées, déposées par les Demanderesses, ont décrit des polymères nouveaux à unités urée, uréthane ou thiourée, ainsi que leurs monomères de départ, qui répondent aux objectifs ci-dessus. Utilisés notamment comme primaire d'adhésion sur métal dans des composites métal / caoutchouc, ces polymères permettent très avantageusement de coller le métal aux matrices de caoutchouc en utilisant ensuite de simples colles textiles telles que des colles « RFL » (résorcinol-formaldéhyde-latex) ou autres compositions adhésives équivalentes, ou encore directement (c'est-à-dire sans emploi de telles colles) à ces matrices de caoutchouc lorsque ces dernières contiennent par exemple des élastomères insaturés fonctionnalisés appropriés tels que des élastomères époxydés. Ainsi peuvent être supprimés notamment les sels de cobalt (ou autres sels métalliques) dans les compositions de caoutchouc destinées à être reliées à des renforts métalliques laitonnés Des adhésifs contenant de la benzoxazine sont décrits dans le document WO2010002872 et sont utilisables dans des applications automobiles, en particulier pour les composants de freins. Le document JP2004083623 décrit une composition adhésive pour coller du métal et des caoutchoucs, cette composition comprenant une résine thermodurcissable à base de benzoxazine en mélange avec une résine epoxy de type novolac. Le document WO2007/064801 décrit une résine thermodurcissable à base de benzoxazine et d'une combinaison de produits d'additions pour une nouvelle composition durcissable. Aucun de ces documents ne décrit des benzoxazines borées.

Poursuivant leurs recherches, les Demanderesses ont trouvé un composé benzoxazine nouveau, utilisable comme monomère pour la synthèse d'une polybenzoxazine, du type thermodurcissable, qui à température ambiante présente les mêmes performances adhésives, vis-à-vis du métal et du caoutchouc, que les polymères précités mais qui présente une fois thermodurcie (réticulée) une stabilité thermique et chimique encore améliorée et dont la microstructure spécifique, en outre, permet très avantageusement d'ajuster la flexibilité de la molécule selon les applications particulières visées.

### 3. BREVE DESCRIPTION DE L'INVENTION

La présente invention concerne une benzoxazine borée répondant à la formule (A) ci-dessous : 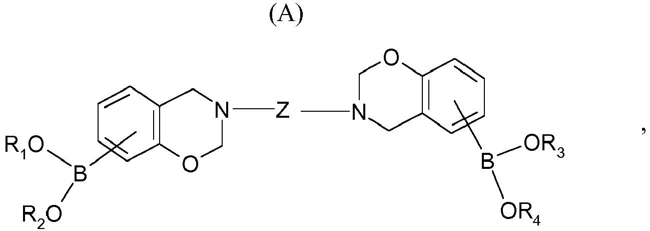 dans laquelle :
- Z représente un groupement de liaison au moins divalent, aliphatique, cycloaliphatique ou aromatique, comportant au moins un atome de carbone et optionnellement au moins un hétéroatome choisi parmi O, S et P ;
- R₁, R₂, R₃ et R₄, identiques ou différents, représentent l'hydrogène ou un alkyle comportant de 1 à 12 atomes de carbone, R₁ et R₂ d'une part, R₃ et R₄ d'autre part pouvant optionnellement former un hétérocycle avec les deux atomes d'oxygène et l'atome de bore auxquels ils sont respectivement liés.

Grâce à cette benzoxazine spécifique, il est possible de préparer des polymères benzoxazine ou « polybenzoxazines » qui ont la capacité remarquable, à haute température, d'ouvrir leurs cycles oxazine et de conduire ainsi à une structure de résine polyphénolique thermodurcissable. Ceci leur confère, comparativement aux autres polymères connus décrits en introduction du présent mémoire, une meilleure stabilité thermique. Sa microstructure spécifique permet enfin, très avantageusement, d'ajuster la flexibilité des polybenzoxazines selon les applications particulières visées.

L'invention concerne également l'utilisation d'un composé conforme à l'invention pour la synthèse d'une polybenzoxazine.

L'invention concerne également tout procédé de synthèse d'une polybenzoxazine par polycondensation d'une benzoxazine borée selon l'invention (à titre de premier monomère), en particulier avec (à titre de second monomère) une benzoxazine bromée répondant à la formule (B) ci-dessous : 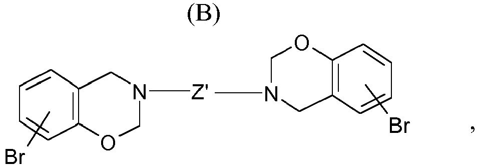 formule (B) dans laquelle Z', identique à ou différent de Z précédemment défini, représente lui-même un groupement de liaison au moins divalent, aliphatique, cycloaliphatique ou aromatique, comportant au moins un atome de carbone et optionnellement au moins un hétéroatome choisi parmi O, S et P.

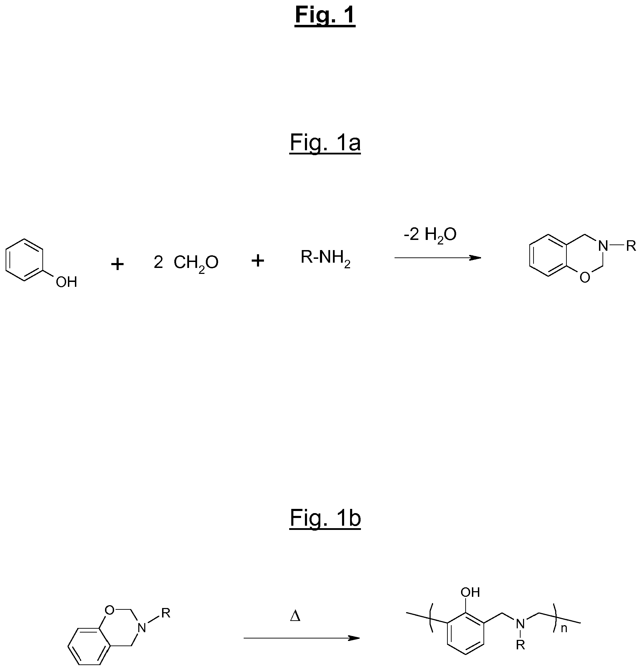

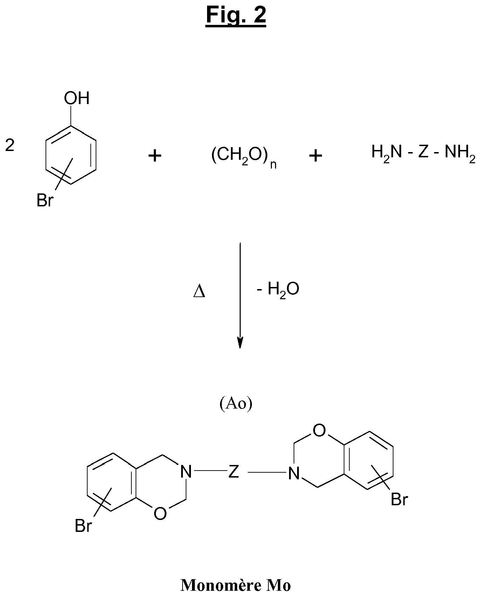

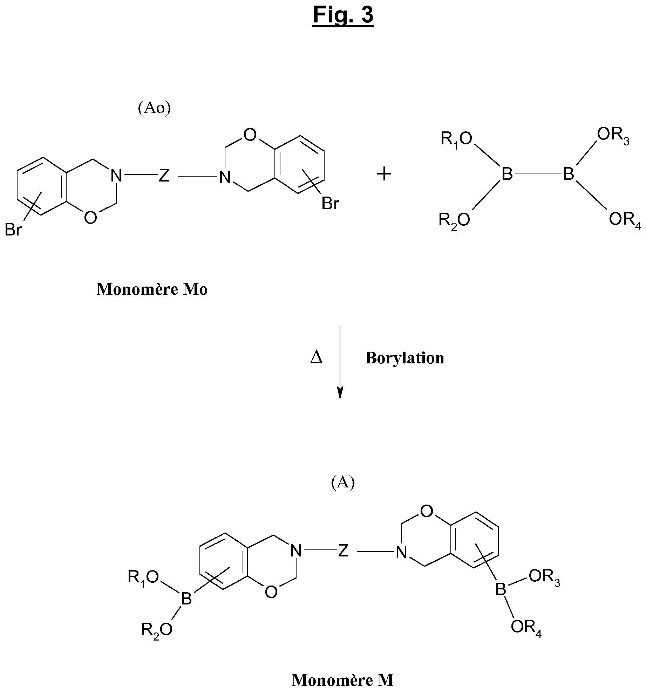

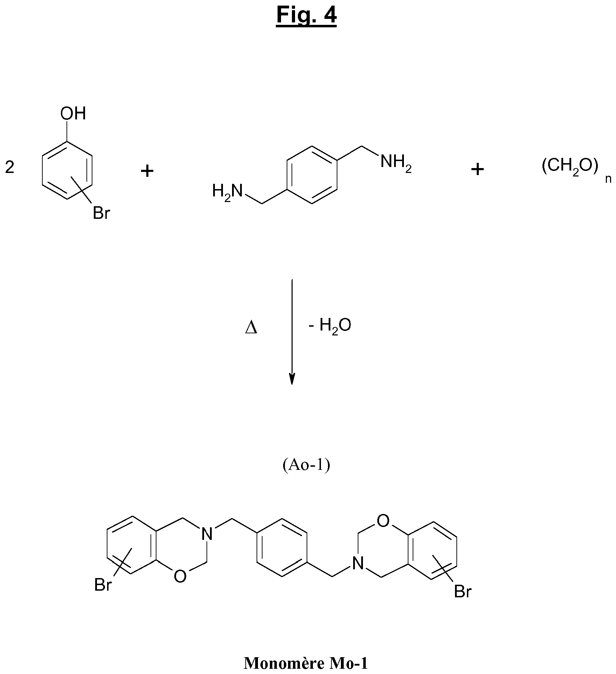

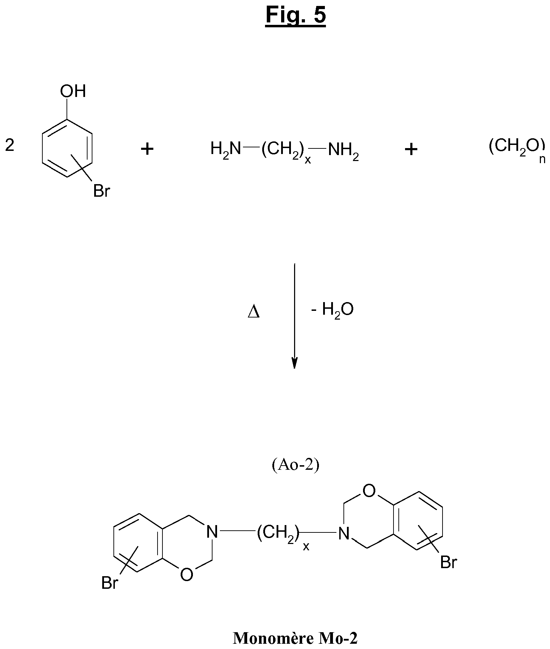

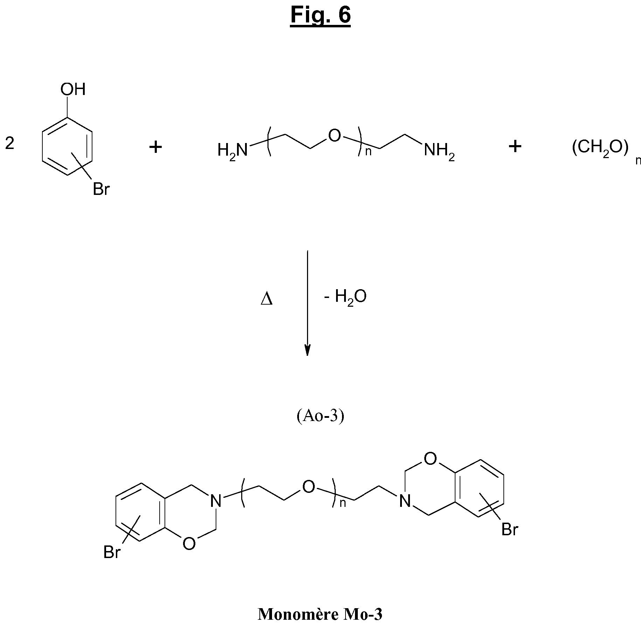

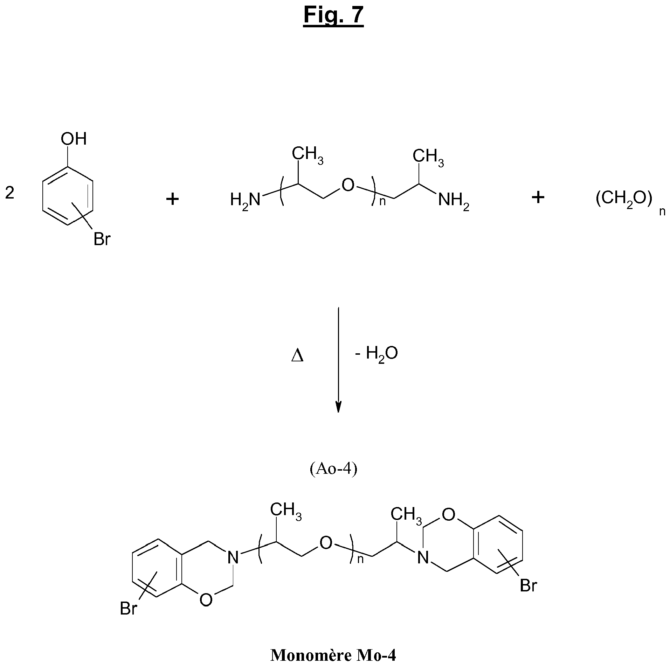

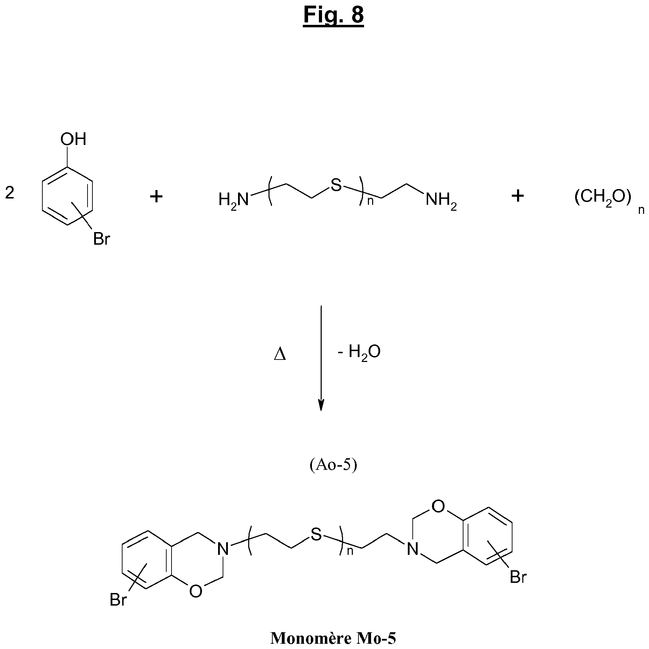

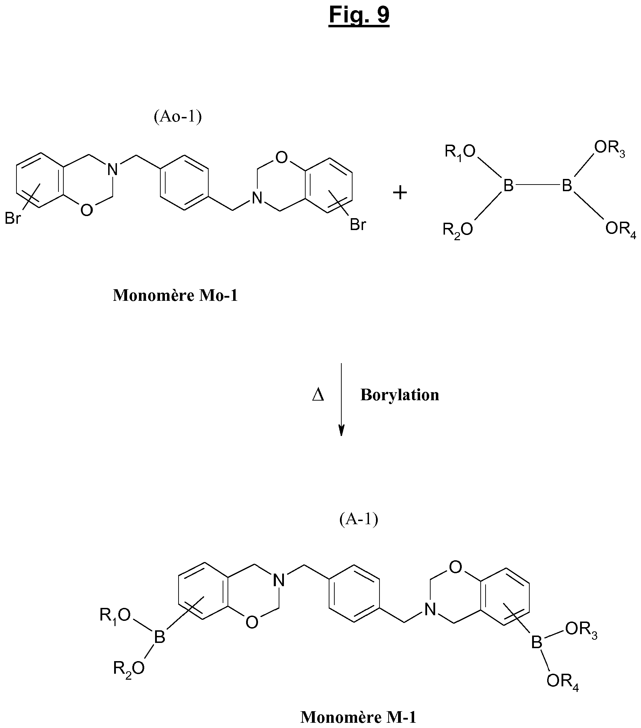

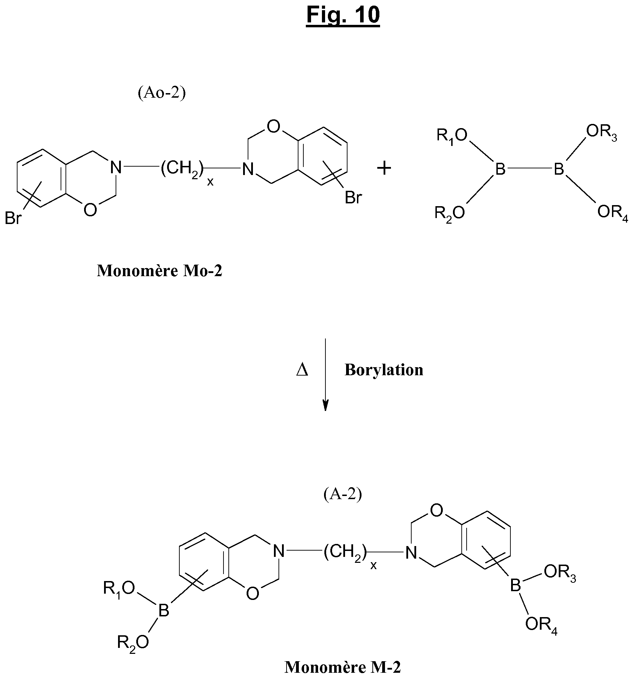

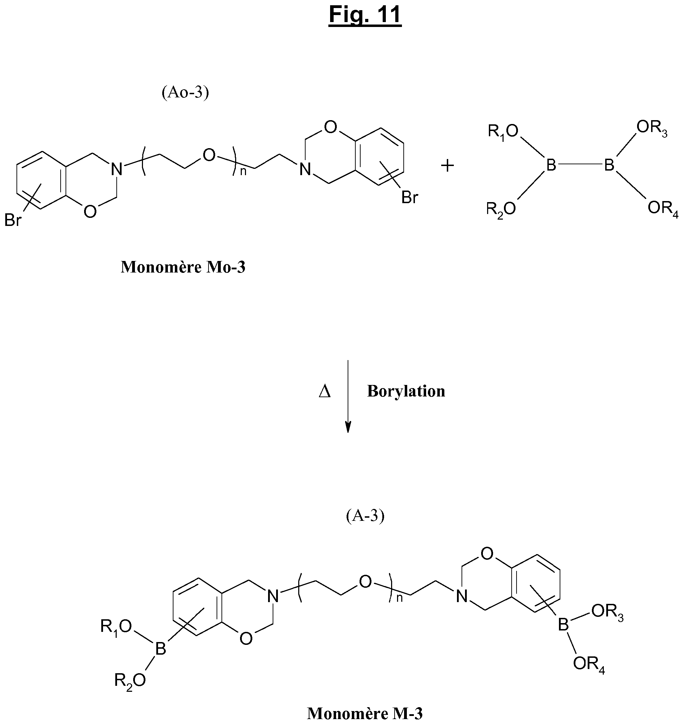

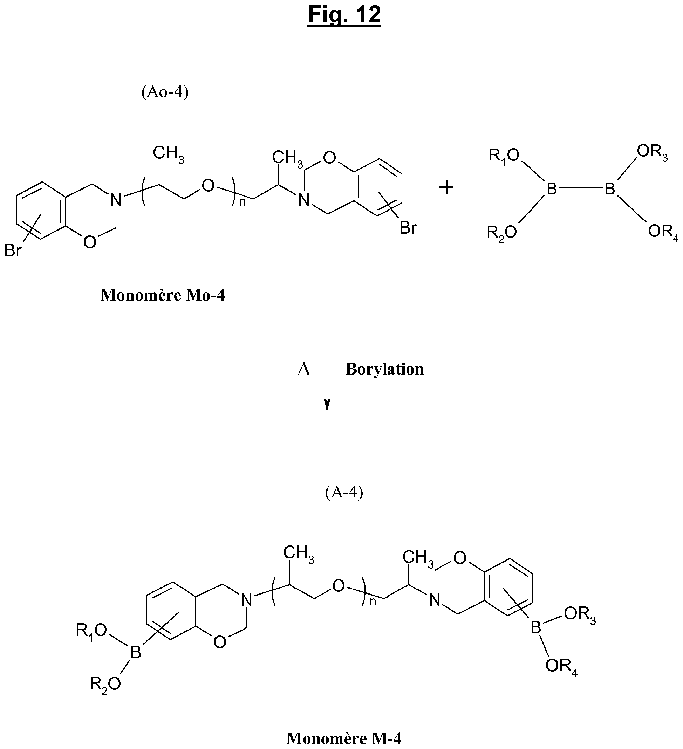

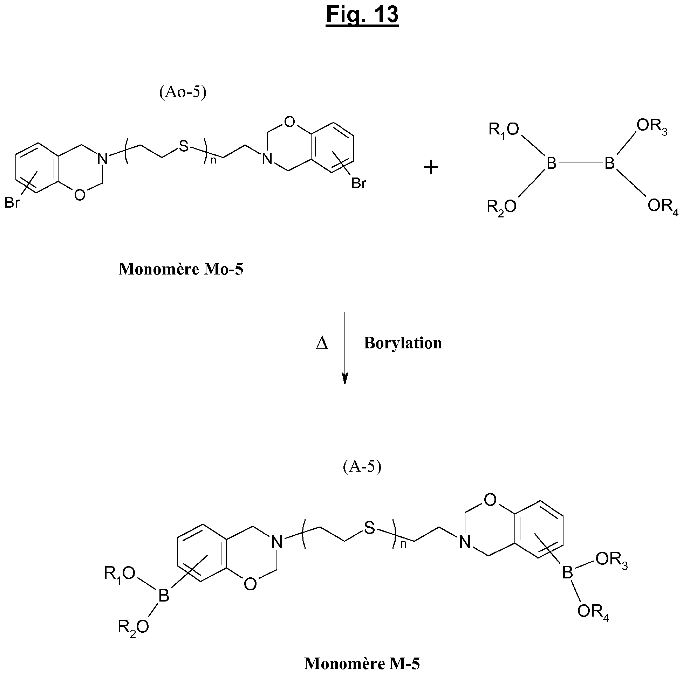

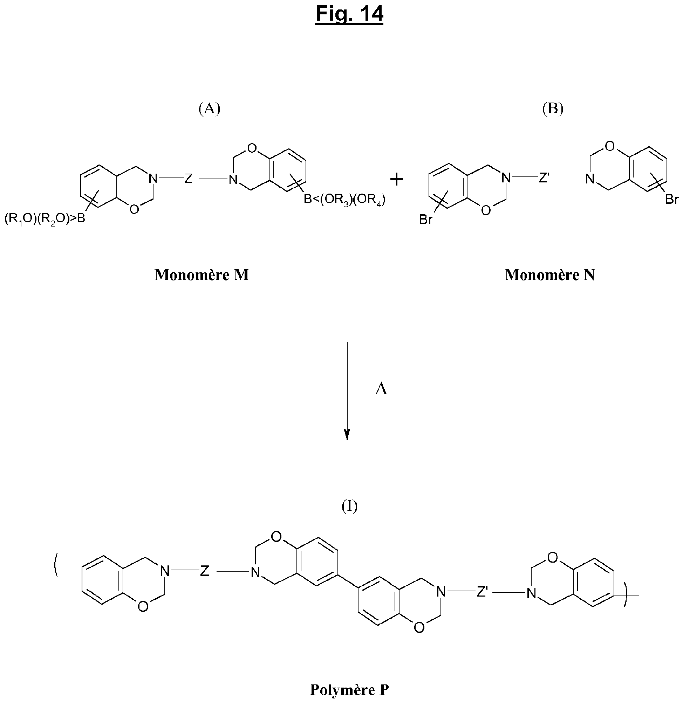

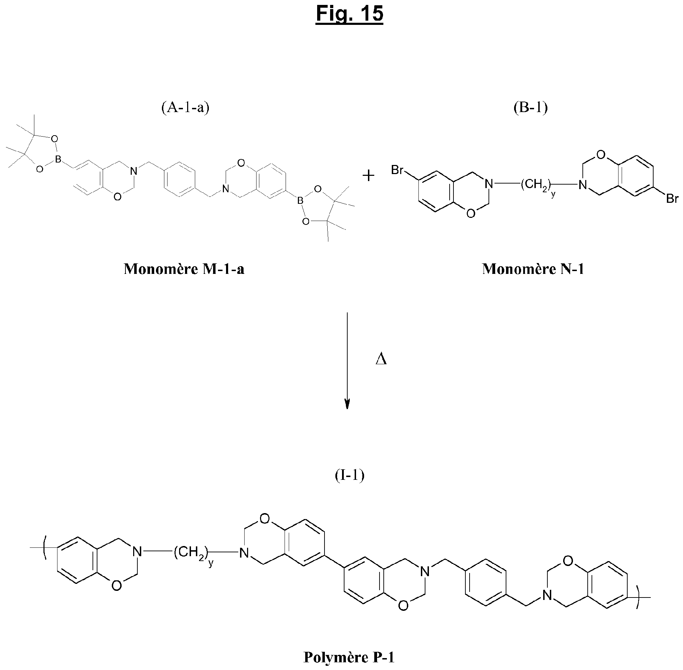

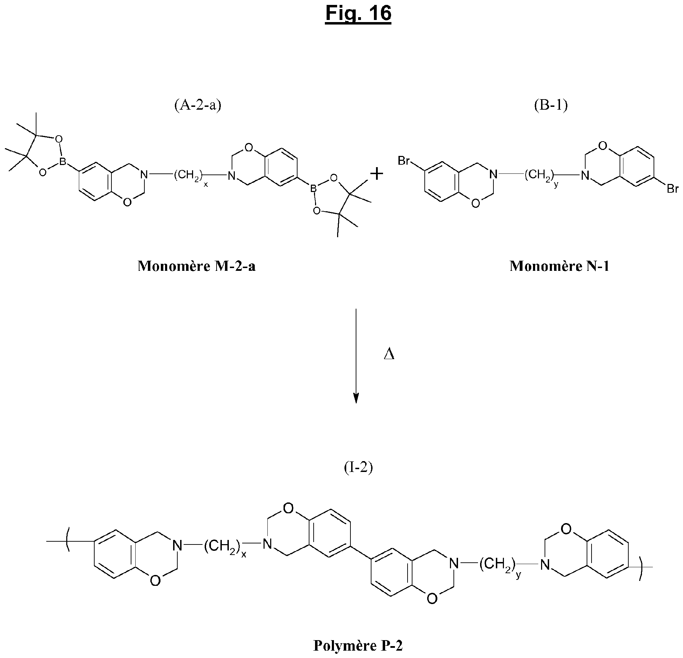

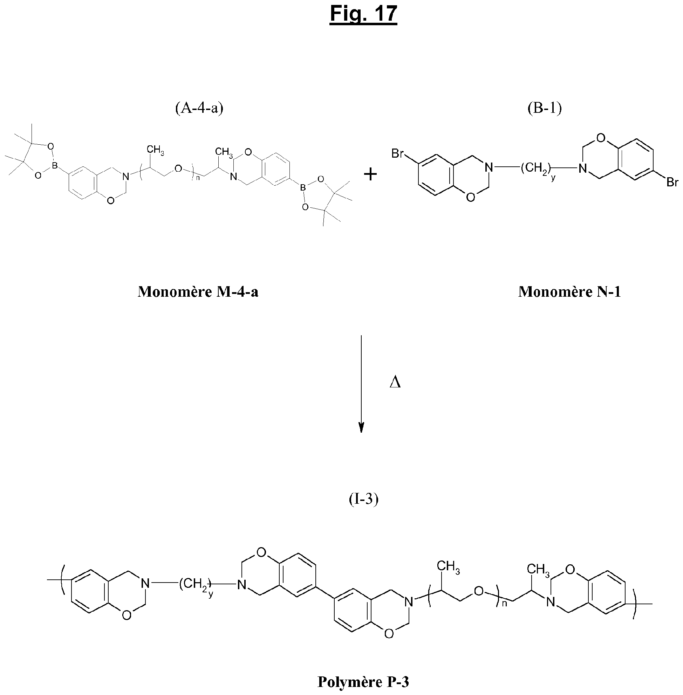

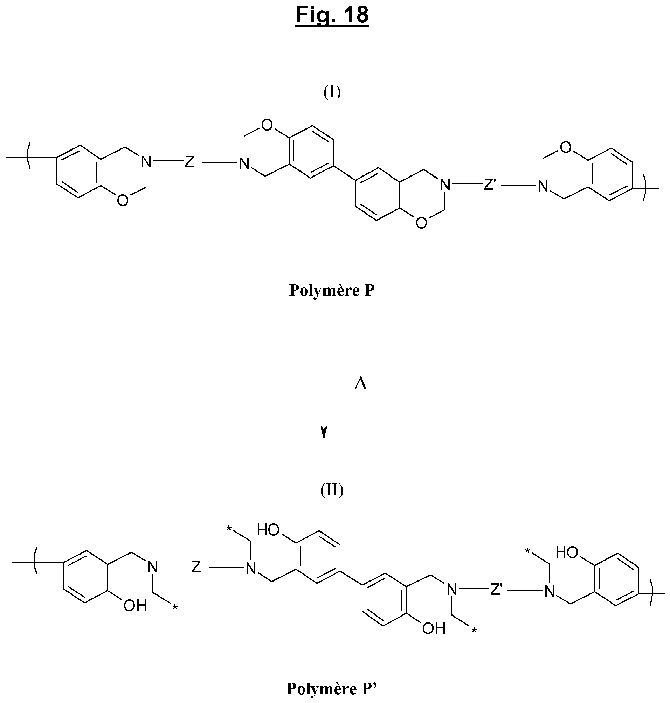

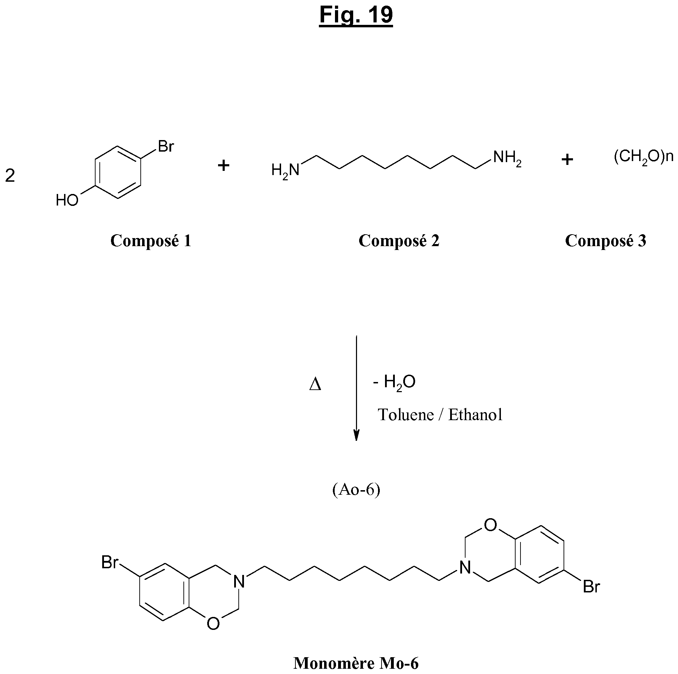

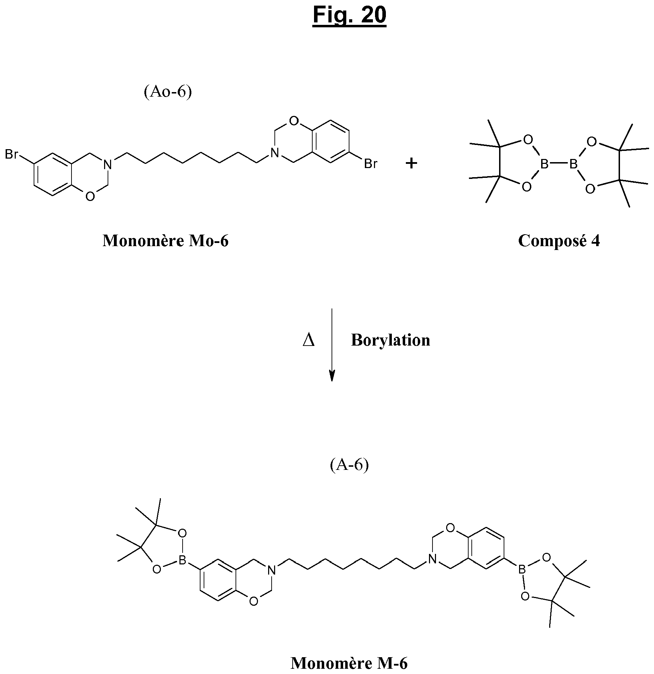

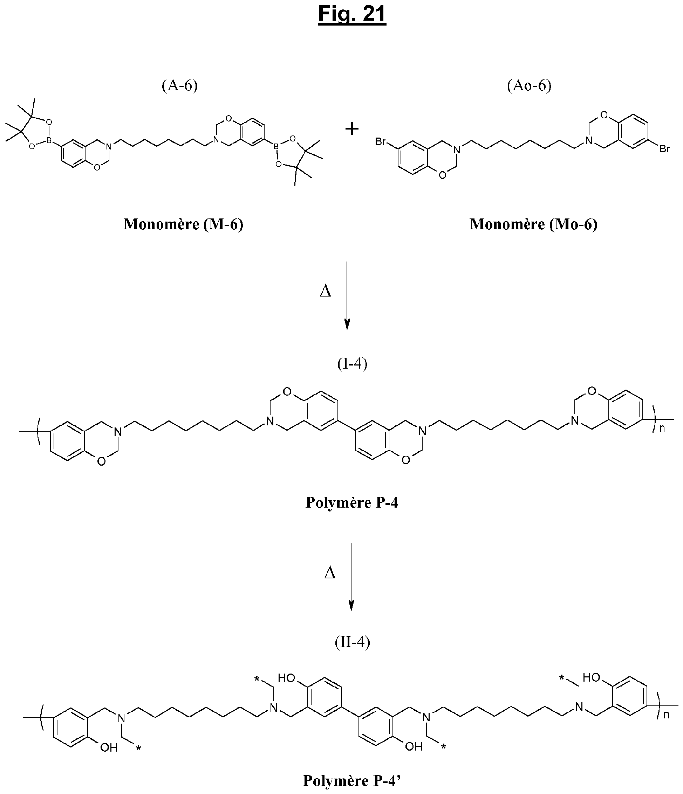

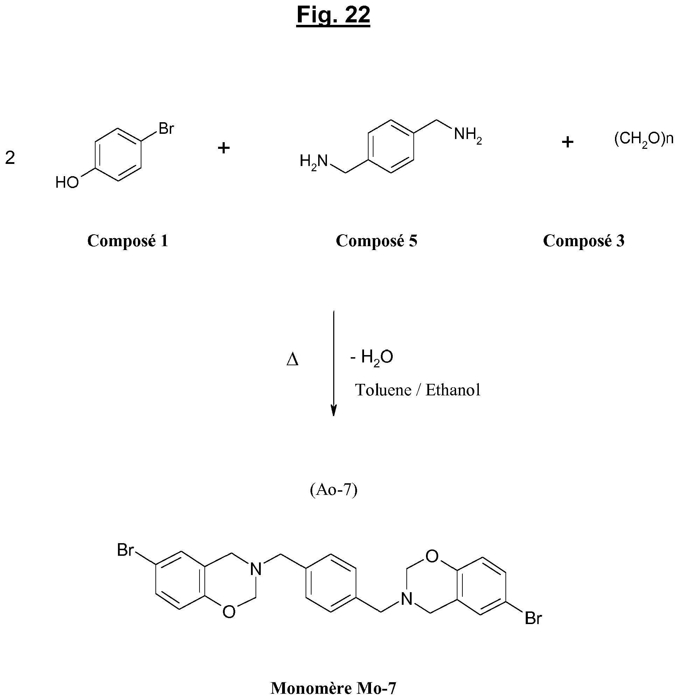

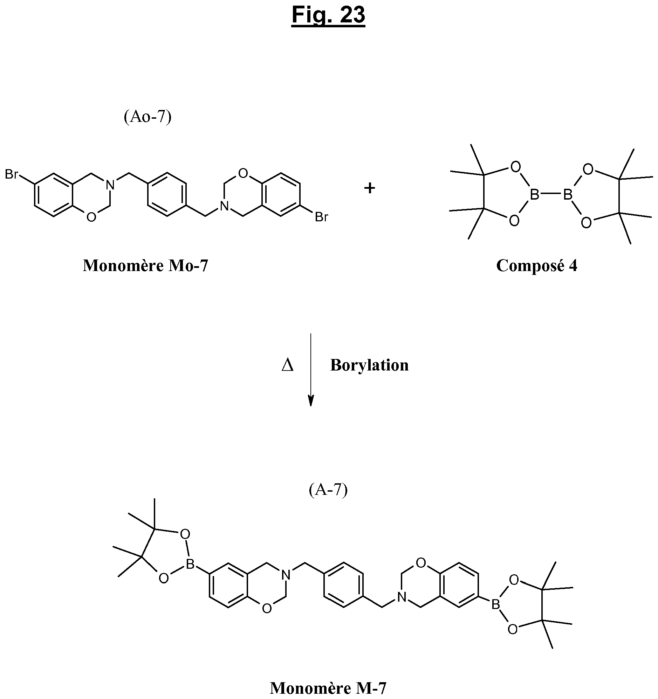

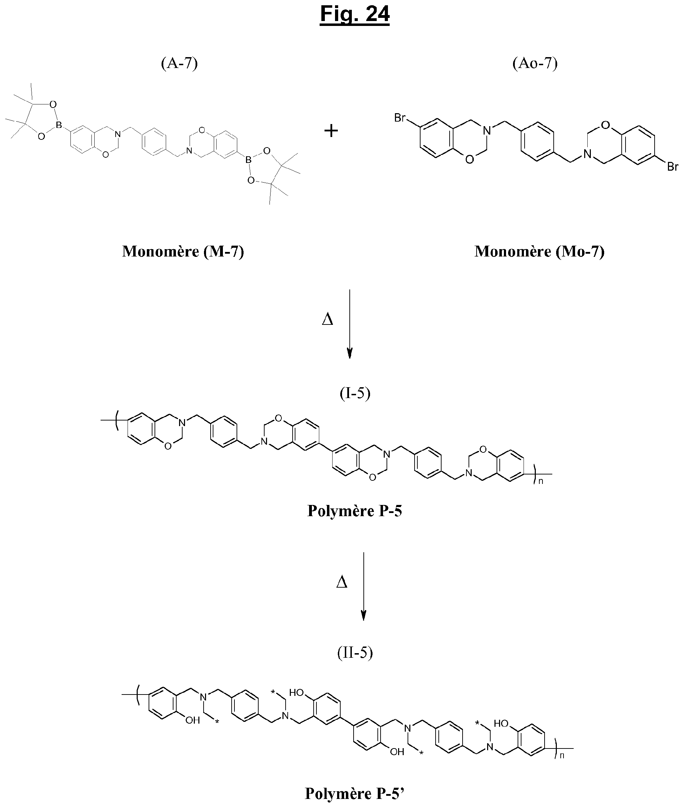

L'invention ainsi que ses avantages seront aisément compris à la lumière de la description détaillée et des exemples de réalisation qui suivent, ainsi que des figures 1 à 24 relatives à ces exemples qui représentent ou schématisent :
- le principe général de synthèse d'un composé benzoxazine à partir de trois composés, phénol, formaldéhyde et amine (R résidu de l'aminé) (Fig. la) ;
- le mécanisme d'ouverture, par apport thermique, du cycle oxazine (« ring-opening ») d'un tel composé benzoxazine (Fig. 1b) ;
- un schéma de synthèse, à partir d'un phénol bromé (Br représentant un atome de brome), de paraformaldéhyde et d'une diamine, d'une benzoxazine bromée de formule (Ao), utilisable comme composé de départ (Monomère noté « Mo ») pour la synthèse d'une benzoxazine borée selon l'invention (Fig. 2) ;
- un schéma de synthèse général d'une benzoxazine borée de formule (A), conforme à l'invention, par une réaction de borylation d'une benzoxazine bromée de formule (Ao) précédente par un composé (ester ou acide) diboronique, cette benzoxazine borée étant utilisable comme monomère de départ (Monomère noté « M ») pour la synthèse d'une polybenzoxazine (Fig. 3) ;
- un schéma de synthèse possible, à partir de phénol bromé, p-formaldéhyde et d'une diamine spécifique du type aromatique, d'une benzoxazine bromée particulière, de formule (Ao-1), utilisable comme composé de départ (Monomère noté Mo-1) pour la synthèse d'une benzoxazine borée particulière conforme à l'invention (Fig. 4) ;
- un autre schéma de synthèse possible, à partir de phénol halogéné, p-formaldéhyde et d'une autre diamine spécifique, cette fois du type aliphatique, d'un autre exemple de benzoxazine bromée particulière, de formule (Ao-2), utilisable comme monomère de départ (Monomère noté Mo-2) pour la synthèse d'un autre exemple de benzoxazine borée conforme à l'invention (Fig. 5) ;
- trois autres schémas de synthèse possibles, à partir de phénol halogéné, p-formaldéhyde et de diamines spécifiques toutes aliphatiques, d'autres exemples de benzoxazines bromées particulières, de formules respectives (Ao-3), (Ao-4) et (Ao-5), toutes utilisables comme monomère de départ (Monomères notés respectivement Mo-3, Mo-4 et Mo-5) pour la synthèse d'autres exemples de benzoxazines borées selon l'invention (Fig. 6, Fig. 7 et Fig. 8) ;
- un schéma de synthèse d'un exemple de benzoxazine borée conforme à l'invention, de formule (A-1), par borylation de la benzoxazine bromée de formule (Ao-1) précédente à l'aide d'un ester ou acide diboronique, cette benzoxazine borée selon l'invention étant utilisable comme monomère de départ (Monomère noté M-1) pour la synthèse d'une polybenzoxazine (Fig. 9) ;
- divers autres schémas de synthèse d'autres exemple de benzoxazines borées conformes à l'invention, de formules respectives (A-2), (A-3), (A-4) et (A-5), par borylation des benzoxazines bromées de formules respectives (Ao-2), (Ao-3), (Ao-4) et (Ao-5) précédentes, à l'aide d'un ester ou acide diboronique, ces benzoxazines borées selon l'invention étant utilisables comme monomères de départ (Monomères notés respectivement M-2, M-3, M-4 et M-5) pour la synthèse de polybenzoxazines (Fig. 10, Fig. 11, Fig. 12 et Fig. 13) ;
- un schéma de synthèse général d'un polymère polybenzoxazine (Polymère noté « P ») par polycondensation de la benzoxazine borée de l'invention de formule (A) (Monomère M) de la figure 3 précédente et d'une autre benzoxazine de formule générique (B) (Monomère noté « N ») du type bromée (Fig. 14) ;
- un schéma de synthèse particulier d'un polymère polybenzoxazine particulier (Polymère noté P-1) à partir d'une benzoxazine borée spécifique selon l'invention de formule (A-1-a) (Monomère M-1-a) et d'une autre benzoxazine de formule générique (B-1) (Monomère noté N-1) du type bromée (Fig. 15) ;
- deux autres schémas de synthèse possibles de polymères polybenzoxazine particuliers (Polymères notés respectivement P-2 et P-3) par polycondensation de benzoxazines borées spécifiques selon l'invention de formules respectives (A-2-a) et (A-4-a) (Monomères M-2-a et M-4-a) avec l'autre benzoxazine de formule générique (B-1) (Monomère N-1) du type bromée (Fig. 16 et Fig. 17) ;
- la polybenzoxazine (Polymère noté ici « P' ») de la figure 14 une fois ses cycles oxazine ouverts après traitement thermique du Polymère P (Fig. 18) ;
- le schéma de synthèse, à partir de phénol bromé (composé 1), p-formaldéhyde (composé 3) et d'une diamine spécifique aliphatique (composé 2), d'une dibenzoxazine bromée particulière de formule (Ao-6) (Monomère noté Mo-6) utilisable pour la synthèse d'une benzoxazine borée conforme à l'invention (Fig. 19) ;
- un schéma de synthèse spécifique d'une benzoxazine borée selon l'invention, de formule (A-6), par borylation de la benzoxazine bromée de formule (Ao-6) précédente à l'aide d'un ester diboronique (bis-pinacol ester de l'acide borique) (composé 4), cette benzoxazine borée selon l'invention étant utilisable comme monomère de départ (Monomère noté M-6) pour la synthèse d'une polybenzoxazine (Fig. 20) ;
- un schéma de synthèse spécifique d'un exemple de polymère polybenzoxazine particulier (Polymère noté P-4) par polycondensation de la benzoxazine borée spécifique précédente selon l'invention de formule (A-6) (Monomère M-6) avec sa benzoxazine équivalente de départ de formule (Ao-6) (Monomère Mo-6) du type bromée, ainsi que cette même polybenzoxazine (Polymère noté P-4') une fois ses cycles oxazine ouverts après traitement thermique du Polymère P-4 (Fig. 21) ;
- un autre schéma de synthèse, à partir de phénol bromé (composé 1), p-formaldéhyde (composé 3) et d'une autre diamine spécifique ici de type aromatique (composé 5), d'une dibenzoxazine bromée particulière de formule (Ao-7) (Monomère noté Mo-7) utilisable pour la synthèse d'une benzoxazine borée conforme à l'invention (Fig. 22) ;
- un schéma de synthèse spécifique d'une benzoxazine borée conforme à l'invention, de formule (A-7), par borylation de la benzoxazine bromée de formule (Ao-7) précédente à l'aide d'un ester diboronique (bis-pinacol ester de l'acide borique) (composé 4), cette benzoxazine borée selon l'invention étant utilisable comme monomère de départ (Monomère noté M-7) pour la synthèse d'une polybenzoxazine (Fig. 23) ;
- enfin, un schéma de synthèse spécifique d'un exemple de polymère polybenzoxazine particulier (Polymère noté P-5) par polycondensation de la benzoxazine borée spécifique précédente selon l'invention de formule (A-7) (Monomère M-7) avec sa benzoxazine équivalente de départ de formule (Ao-7) (Monomère Mo-7) du type bromée, ainsi que cette même polybenzoxazine (Polymère noté P-5') une fois ses cycles oxazine ouverts après traitement thermique du Polymère P-5 (Fig. 24).

### 4. DESCRIPTION DETAILLEE DE L'INVENTION

On rappellera tout d'abord que les benzoxazines sont des composés de formule générale :

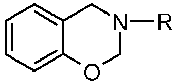

La figure la annexée rappelle le principe général de synthèse d'une benzoxazine, ici à partir (réaction de condensation) d'une molécule de phénol, de deux molécules de formaldéhyde et d'une amine (R désignant le résidu de l'amine), avec élimination de deux molécules d'eau.

La figure 1b rappelle quant à elle le mécanisme d'ouverture (« *ring-opening* ») du cycle oxazine d'un tel composé lors d'un apport thermique (représenté par le symbole Δ).

De nombreux composés ou monomères benzoxazines peuvent être ainsi synthétisés en utilisant divers phénols et amines selon leurs types de substituants. Ces groupes substituants peuvent fournir ensuite des sites polymérisables et permettre la synthèse de divers polymères benzoxazine (ou polybenzoxazines).

Benzoxazines et polybenzoxazines qui en sont issues sont des produits aujourd'hui bien connus de l'homme du métier ; pour ne citer que quelques exemples de publication, on peut mentionner les articles « Polybenzoxazines - New high performance thermosetting resins : synthesis and properties » ; N.N. Ghosh et al., Prog. Polym. Sci. 32 (2007), 1344-1391, ou « Recent Advancement on Polybenzoxazine - A newly Developed High Performance Thermoset », Y. Yaggi et al., J. Polym. Sci. Part A: Polym. Chem. : Vol. 47 (2009), 5565-5576, ainsi que par exemple les brevets ou demandes de brevet US 5 543 516, WO 2013/148408.

Comme expliqué en détail dans les documents ci-dessus, les polybenzoxazines ont la capacité remarquable, à haute température (par exemple, typiquement supérieure à 150°C voire à 200°C selon leur microstructure particulière), d'ouvrir leurs cycles oxazine et de conduire ainsi à des structures de résines polyphénoliques thermodurcissables.

La benzoxazine spécifique de l'invention, dénommée « Monomère M » dans la présente demande, est du type borée ; elle répond à la formule générique (A) qui suit (le symbole B représentant bien entendu un atome de Bore) : 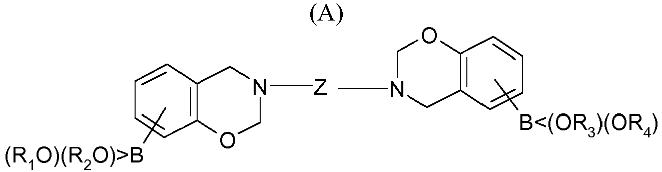 dans laquelle :
- Z représente un groupement de liaison au moins divalent, aliphatique, cycloaliphatique ou aromatique, comportant au moins un atome de carbone et optionnellement au moins un hétéroatome choisi parmi O, S et P ;
- R₁, R₂, R₃ et R₄, identiques ou différents, représentent l'hydrogène ou un alkyle comportant de 1 à 12 atomes de carbone.

Dans la formule (A) ci-dessus, R₁ et R₂ d'une part, R₃ et R₄ d'autre part peuvent optionnellement former un hétérocycle avec les deux atomes d'oxygène et l'atome de bore auxquels ils sont respectivement liés ; on comprendra aisément que, dans un tel cas, ces alkyles sont plus précisément des cycloalkyles.

La figure 3 annexée en donne le schéma de synthèse général par réaction dite de « borylation » d'une benzoxazine bromée de départ formule (Ao) par un composé (ester ou acide) diboronique, la figure 2 reproduisant quant à elle le procédé de synthèse de cette benzoxazine de départ de formule (Ao), sous apport thermique et avec élimination d'eau, à partir d'un phénol halogéné, de p-formaldéhyde et d'une diamine.

Dans la formule (A) ci-dessus, Z représente un groupement de liaison (espaceur ou *« spacer »*) au moins divalent c'est-à-dire qu'il pourrait comporter plus de deux liaisons covalentes, par exemple trois ou quatre liaisons covalentes. De préférence, Z est divalent, c'est-à-dire ne comporte que deux liaisons covalentes.

Z peut être aliphatique, cycloaliphatique ou aromatique. Ce groupement Z, pouvant être éthyléniquement saturé ou insaturé, comporte par définition au moins un (c'est-à-dire un ou plusieurs) atome de carbone, et optionnellement au moins un (c'est-à-dire un ou plusieurs) hétéroatome choisi parmi O (oxygène), S (soufre) et P (phosphore).

Selon un mode de réalisation préférentiel de l'invention, Z comporte au moins un groupement aromatique comportant 6 à 30, de préférence 6 à 20 atomes de carbone (et optionnellement au moins un hétéroatome choisi parmi O, S et P).

Dans un tel cas, le composé de l'invention répond en particulier à la formule (A-1) ci-dessous :

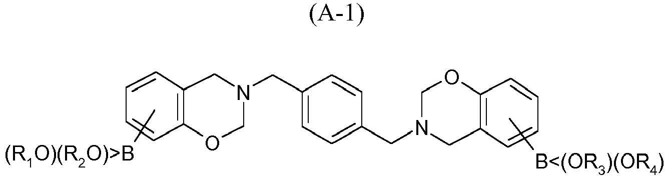

Selon un autre mode de réalisation préférentiel de l'invention, Z représente un groupement aliphatique comportant 1 à 20, de préférence 1 à 16 atomes de carbone, ou un groupement cycloaliphatique comportant 3 à 20, de préférence 3 à 16 atomes de carbone (et optionnellement au moins un hétéroatome choisi parmi O, S et P).

Plus préférentiellement, Z représente un groupement ou enchaînement (poly)alkylène (ou alkylidène) comportant 1 à 20, de préférence 1 à 16 atomes de carbone (et optionnellement au moins un hétéroatome choisi parmi O, S et P). Plus préférentiellement encore, Z représente un enchaînement (poly)alkylène comportant de 1 à 12 atomes de carbone, et optionnellement au moins un hétéroatome choisi parmi O et S.

Dans un tel cas, le composé de l'invention répond plus préférentiellement à l'une des formules (A-2) à (A-5) ci-dessous :

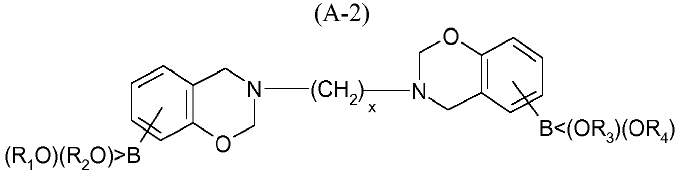

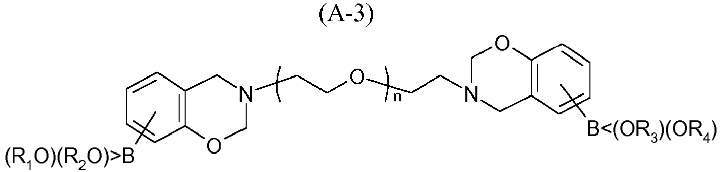

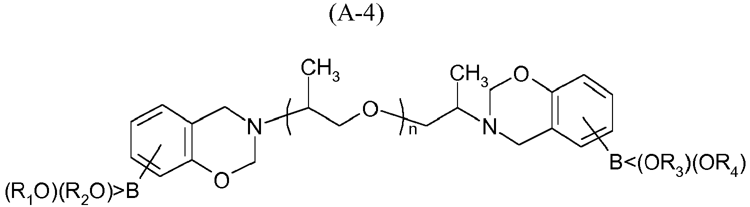

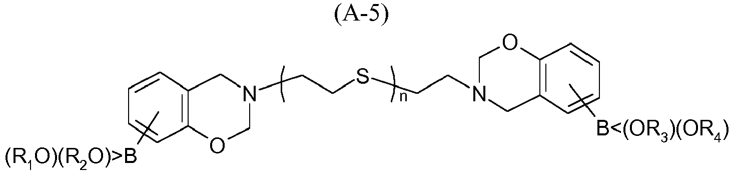

Dans la formule (A) précédente, et en particulier dans les formules (A-1) à (A-5) ci-dessus, les symboles R₁, R₂, R₃ et R₄, identiques ou différents, représentent de préférence un alkyle comportant 1 à 12 atomes de carbone, plus préférentiellement un alkyle comportant 1 à 8, en particulier 1 à 6 atomes de carbone, ces alkyles pouvant optionnellement former un hétérocycle avec les deux atomes d'oxygène et l'atome de bore auxquels ils sont respectivement liés.

Encore plus préférentiellement, R₁ et R₂ d'une part, R₃ et R₄ d'autre part forment un hétérocycle avec les deux atomes d'oxygène et l'atome de bore auxquels ils sont respectivement liés.

Cet hétérocycle répond plus préférentiellement à l'une des trois formules (a), (b) ou (c) qui suivent :

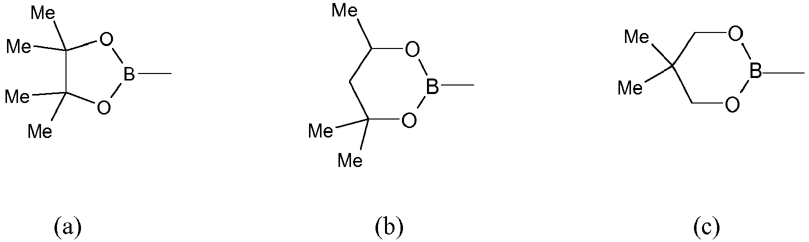

Cet hétérocycle répond encore plus préférentiellement à la formule (a) ci-dessus.

Selon un autre mode de réalisation plus particulier de l'invention, l'atome de bore porté par chaque noyau benzénique est situé en position para de l'oxygène du cycle oxazine.

Ainsi, selon des modes de réalisation particulièrement préférentiels de l'invention, la benzoxazine borée de l'invention répond à l'une des formules (A-1-a) à (A-5-a) ci-dessous :

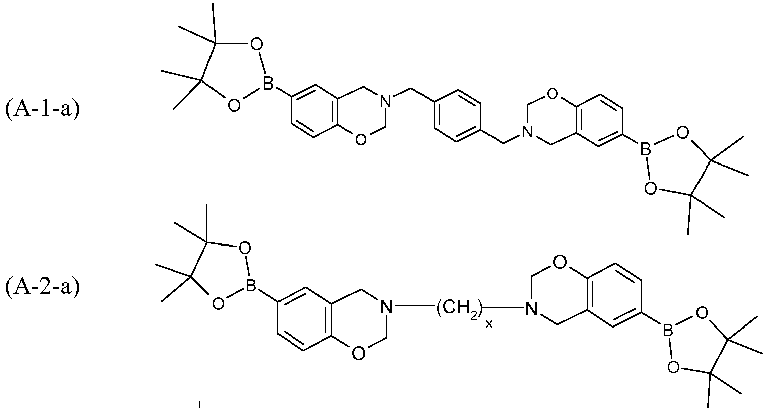

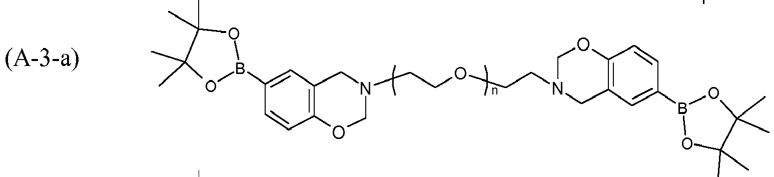

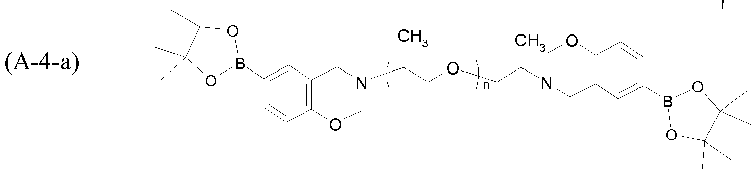

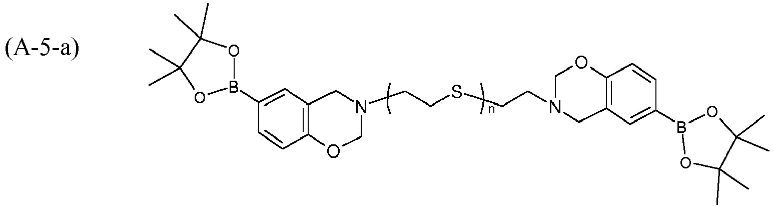

Dans les formules (A-2) à (A-5) supra, en particulier dans les formules (A-2-a) à (A-5-a) ci-dessus, les symboles « x » et « n » représentent un nombre entier, préférentiellement un nombre entier tel que Z (ici, aliphatique) comporte 1 à 20, plus préférentiellement 1 à 16 atomes de carbone (et optionnellement au moins un hétéroatome choisi parmi O, S et P).

Selon un mode de réalisation encore plus particulièrement préférentiel de l'invention, la benzoxazine borée de l'invention répond à la formule (A-4-a) ci-dessus.

Chaque noyau benzénique des deux cycles oxazine du Monomère M de l'invention est donc porteur d'un atome de bore. En outre, dans ce monomère de formule (A), un ou plusieurs atomes d'hydrogène d'au moins un ou de chaque noyau benzénique des deux cycles oxazine peuvent être substitués (ou non) par différents substituants, par exemple par des groupes fonctionnels susceptibles de favoriser encore l'adhésion du polymère au métal et/ou au caoutchouc.

Comme dit précédemment, selon un premier mode de réalisation particulièrement préférentiel, Z comporte un groupement aromatique comportant 6 à 30, plus préférentiellement 6 à 20 atomes de carbone.

Ainsi, la figure 4 illustre un schéma de synthèse possible, sous apport thermique et avec élimination d'eau, à partir de phénol bromé, p-formaldéhyde et d'une diamine spécifique du type aromatique (p-xylylène diamine), d'une benzoxazine bromée particulière, de formule (Ao-1), utilisable comme composé de départ (Monomère noté Mo-1) pour la synthèse ultérieure d'une benzoxazine borée conforme à l'invention répondant au premier mode de réalisation particulièrement préférentiel énoncé ci-dessus.

Selon un autre mode de réalisation particulièrement préférentiel, Z représente un enchaînement (poly)alkylène (ou alkylidène) comportant 1 à 16, plus particulièrement 1 à 12 atomes de carbone, un tel enchaînement pouvant être interrompu optionnellement par au moins un hétéroatome choisi parmi O et S.

Ainsi, la figure 5 illustre un schéma de synthèse possible, sous apport thermique et avec élimination d'eau, à partir du phénol bromé, p-formaldéhyde et d'une autre diamine spécifique, cette fois du type aliphatique (polyéthylène diamine), d'un autre exemple de benzoxazine bromée particulière, de formule (Ao-2), utilisable comme monomère de départ (Monomère noté Mo-2) pour la synthèse d'une benzoxazine borée conforme à l'invention répondant à l'autre mode de réalisation particulièrement préférentiel énoncé ci-dessus. On note que Z représente ici un groupement -(CH₂)ₓ- dans lequel le symbole « x » représente un nombre entier, de préférence de 1 à 12. Une telle synthèse sera décrite plus en détail dans les exemples de réalisation qui suivent (Fig. 19).

Les figures 6, 7 et 8 illustrent trois autres schémas de synthèse possibles, toujours à partir de phénol halogéné et de paraformaldéhyde d'une part, et d'autre part de différentes diamines spécifiques, toutes du type aliphatiques, d'autres exemples de benzoxazines bromées particulières, de formules respectives (Ao-3), (Ao-4) et (Ao-5), toutes utilisables comme monomère de départ (Monomères notés Mo-3, Mo-4 et Mo-5) pour la synthèse ultérieure d'autres exemples de benzoxazines borées selon l'invention, de formules respectives (A-3), (A-4) et (A-5). Dans ces formules, le symbole « n » représente un nombre entier, de préférence un nombre entier tel que Z (ici aliphatique) comporte 1 à 20, plus préférentiellement 1 à 16 atomes de carbone (et optionnellement au moins un hétéroatome choisi parmi O, S et P).

A la figure 6, la répétition des motifs [-CH₂-CH₂-O-] (polyéthylène oxyde) sur le groupement de liaison Z est susceptible de conduire à des polybenzoxazines de haute cristallinité, tandis qu'à la figure 7, la présence des groupes méthyle (polypropylène oxyde) sur Z permet de diminuer la réactivité des deux groupements terminaux amine et de conduire à des polybenzoxazines de moindre cristallinité. A la figure 8, la présence sur le spacer Z de l'atome (hétéroatome) de soufre dans les motifs récurrents [-CH₂-CH₂-S-] (polyéthylène thioéther), est susceptible d'améliorer encore l'adhésion au métal de la polybenzoxazine. Ainsi, on peut voir que la structure du groupement Z peut être modifiée largement dans le but de moduler les propriétés de la benzoxazine borée de l'invention et celles du polymère (polybenzoxazine) final. Ceci constitue un avantage majeur de la présente invention.

La figure 9 illustre maintenant un schéma de synthèse d'un exemple de benzoxazine borée conforme à l'invention, de formule particulière (A-1), par borylation de la benzoxazine bromée de formule (Ao-1) précédente à l'aide d'un ester ou acide diboronique, cette benzoxazine borée selon l'invention étant utilisable comme monomère de départ (Monomère noté M-1) pour la synthèse d'une polybenzoxazine selon le procédé de l'invention.

Les figures 10, 11, 12 et 13 donnent divers autres schémas de synthèse d'autres exemples de benzoxazines borées conformes à l'invention, de formules respectives (A-2), (A-3), (A-4) et (A-5), par borylation des benzoxazines bromées de formules respectives (Ao-2), (Ao-3), (Ao-4) et (Ao-5) précédentes, à l'aide d'un ester ou acide diboronique, ces benzoxazines borées selon l'invention étant utilisables comme monomères de départ (Monomères notés respectivement M-2, M-3, M-4 et M-5) pour la synthèse d'autres polybenzoxazines selon le procédé de l'invention.

La benzoxazine borée conforme à l'invention de formule (A) précédemment décrite est particulièrement destinée (à titre de premier Monomère « M ») à la synthèse d'une polybenzoxazine par polycondensation, en particulier par polycondensation avec au moins (à titre de deuxième Monomère « N ») une autre benzoxazine, du type bromée, ayant pour formule (Br représentant bien entendu un atome de brome) : 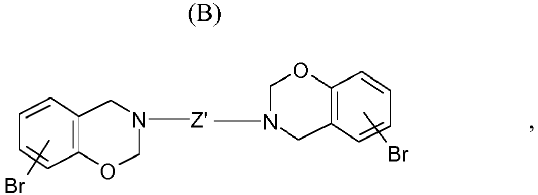 formule (B) dans laquelle Z', identique à ou différent de Z précédemment défini, représente un groupement de liaison au moins divalent, aliphatique, cycloaliphatique ou aromatique, comportant au moins un atome de carbone et optionnellement au moins un hétéroatome choisi parmi O, S et P.

La figure 14 représente un schéma général de synthèse d'une polybenzoxazine (Polymère noté « P »), par polycondensation de la benzoxazine borée selon l'invention de formule (A) de la Fig. 3 (Monomère M), à titre de premier monomère, avec, à titre de deuxième monomère, l'autre monomère de formule générique (B) qui est ici une benzoxazine (Monomère noté « N ») du type bromée.

En outre, comme pour la formule (A) précédente, un ou plusieurs atomes d'hydrogène d'au moins un ou de chaque noyau benzénique des deux cycles oxazine de la formule (B) ci-dessus pourraient être substitués par un seul ou plusieurs substituants, identiques ou différents, par exemple par des groupes fonctionnels susceptibles de favoriser l'adhésion du polymère au métal et/ou au caoutchouc.

Z', identique à ou différent de Z, a la même définition générale et les mêmes définitions préférentielles que celles indiquées précédemment pour Z.

En particulier, selon un premier mode de réalisation particulièrement préférentiel, Z' comporte un groupement aromatique comportant 6 à 30, plus préférentiellement 6 à 20 atomes de carbone (et optionnellement au moins un hétéroatome choisi parmi O, S et P).

Selon un autre mode de réalisation particulièrement préférentiel, Z' représente un enchaînement (poly)alkylène comportant 1 à 20, plus particulièrement 1 à 16, notamment 1 à 12 atomes de carbone, et optionnellement au moins un hétéroatome choisi parmi O et S.

Ainsi, selon un mode de réalisation particulièrement préférentiel de l'invention, la benzoxazine bromée utilisable (comme monomère « N ») répond à l'une des formules ci-dessous (dans lesquelles « x » et « n » ont déjà été définis supra) :

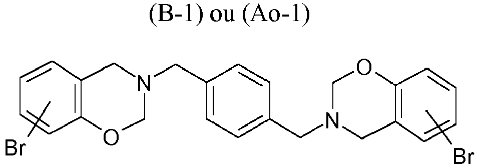

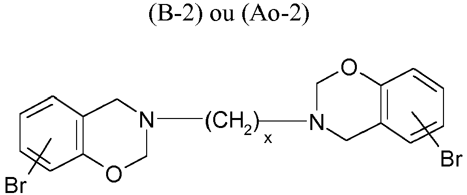

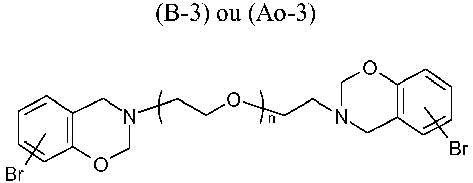

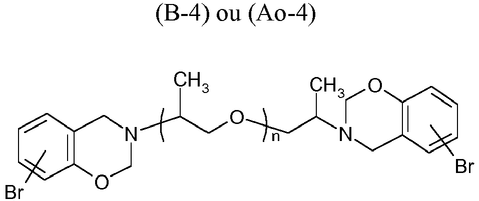

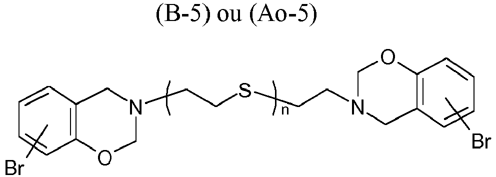

Lorsque Z (formule A) et Z' (formule B) sont identiques, on notera que les formules (B-1) à (B-5) ci-dessus sont bien entendu équivalentes, respectivement, aux formules (Ao-1) à (Ao-5) précédentes, celles précisément des monomères de départ (respectivement Mo-1 à Mo-5) servant à la synthèse des benzoxazines borées de l'invention de formules (A-1) à (A-5) précédemment décrites.

En d'autres termes, dans le procédé conforme à l'invention, ce qui en constitue un mode de réalisation avantageux, les monomères de départ utilisés pour la synthèse des benzoxazines borées de l'invention, peuvent être également utilisables à titre de deuxième monomère pour la synthèse de polybenzoxazines.

Selon un autre mode de réalisation préférentiel de l'invention, dans la formule générale (B) et les formules plus particulières (B-1) à (B-5) ci-dessus, l'atome de brome porté par chaque noyau benzénique des deux cycles oxazine est situé en position para de l'oxygène du cycle oxazine.

La figure 15 reproduit à titre d'exemple un schéma de synthèse particulier d'un polymère polybenzoxazine particulier (Polymère noté P-1) à partir de la benzoxazine borée spécifique selon l'invention de formule (A-1-a) (Monomère M-1-a) et d'une autre benzoxazine de formule générique (B-1) (Monomère noté N-1) du type bromée. On note que Z' représente ici un groupement -(CH₂)_{y}- dans lequel le symbole « y » représente un nombre entier, de préférence de 1 à 12. Une telle synthèse sera décrite plus en détail dans les exemples de réalisation qui suivent.

Les figures 16 et 17 reproduisent à titre d'exemples deux autres schémas de synthèse possibles de polymères polybenzoxazine particuliers (Polymères notés respectivement P-2 et P-3) à partir de benzoxazines borées spécifiques selon l'invention de formules respectives (A-2-a) et (A-4-a) (Monomères M-2-a et M-4-a) et de cette autre benzoxazine de formule générique (B-1) (Monomère noté N-1) du type bromée.

Comme reproduit à la figure 18, la benzoxazine conforme à l'invention de formule (A) est donc particulièrement destinée à la synthèse d'une polybenzoxazine (Polymère noté « P ») comportant des unités structurelles récurrentes comportent au moins un motif répondant aux formule (I) (avant ouverture des cycles oxazine) ou formule (II) (après ouverture des cycles) ci-dessous : 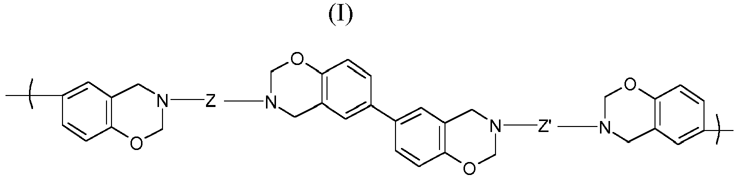 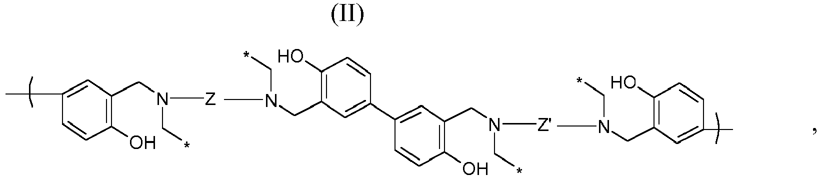 formules (I) et (II) dans lesquelles Z et Z' ont les définitions principales et les définitions préférentielles déjà données précédemment pour la benzoxazine borée de l'invention et sa benzoxazine bromée de départ.

Par polymère doit être entendu ici tout homopolymère ou copolymère, notamment copolymère à blocs, avec des unités structurelles récurrentes comportent au moins un motif de formule (I) ou (II) ci-dessus ; ce polymère peut bien entendu comporter à la fois des motifs de formule (I) et des motifs de formule (II).

Dans la formule (II) ci-dessus, l'homme du métier comprendra immédiatement que les deux symboles « * » (identiques ou différents) représentent un rattachement quelconque du motif à un atome de carbone ou à un hétéroatome (choisi de préférence parmi O, S, N et P), rattachement ou liaison résultant de l'ouverture des cycles oxazine.

Les figures 19, 20 et 21 reproduisent des exemples de synthèse particuliers, successivement d'une dibenzoxazine bromée spécifique, puis d'une benzoxazine borée conforme à l'invention à partir de cette dibenzoxazine bromée, enfin d'un polymère (polybenzoxazine) par polycondensation de ces deux benzoxazines (bromée et borée).

Plus précisément, la figure 19 reproduit le schéma de synthèse, sous apport thermique et avec élimination d'eau, à partir de phénol bromé (composé 1), p-formaldéhyde (composé 3) et d'une diamine spécifique aliphatique (composé 2), d'une dibenzoxazine bromée particulière de formule (Ao-6) (Monomère noté Mo-6) qui va être utilisée pour la synthèse d'une benzoxazine borée conforme à l'invention (benzoxazine de la figure 20 qui suit).

La figure 20 donne le schéma de synthèse spécifique d'une benzoxazine borée conforme à l'invention, de formule (A-6), par borylation de la benzoxazine bromée de formule (Ao-6) précédente à l'aide d'un ester diboronique (bis-pinacol ester de l'acide borique) (composé 4). On note que la formule (A-6) de cet exemple correspond à la formule (A-2-a) précédente dans laquelle « x » est égal à 8.

Finalement, la figure 21 décrit le mode de synthèse spécifique d'un exemple de polymère polybenzoxazine particulier (Polymère noté P-4), par polycondensation de la benzoxazine borée précédente selon l'invention de formule (A-6) (Monomère M-6) avec, à titre de second monomère, sa benzoxazine équivalente de départ de formule (Ao-6) (Monomère Mo-6) du type bromée. Dans cet exemple, on note en particulier que, selon un mode de réalisation préférentiel de l'invention déjà décrit, l'atome de bore dans le Monomère M-6, tout comme l'atome de brome dans le Monomère Mo-6, sont chacun situés en position para de l'oxygène de chaque cycle oxazine.

Cette polybenzoxazine P-4, ou plus exactement au moins une partie de ses unités récurrentes, a été représentée à la figure 21, avant (Formule (I-4) ; Polymère P-4) et après (Formule (II-4) ; Polymère P-4') ouverture de ses cycles oxazine suite à un apport thermique suffisant.

Pour terminer cette description détaillée de l'invention, les figures 22, 23 et 24 reproduisent d'autres exemples de synthèse particuliers, successivement d'une autre dibenzoxazine bromée spécifique, puis d'une benzoxazine borée conforme à l'invention à partir de cette dibenzoxazine bromée, enfin d'un polymère (polybenzoxazine) par polycondensation de ces deux benzoxazines (bromée et borée).

Plus précisément, la figure 22 reproduit le schéma de synthèse, à partir de phénol bromé (composé 1), p-formaldéhyde (composé 3) et d'une autre diamine spécifique ici de type aromatique (composé 5), d'une dibenzoxazine bromée particulière de formule (Ao-7) (Monomère noté Mo-7) utilisable pour la synthèse d'une benzoxazine borée conforme à l'invention (benzoxazine de la figure 23 qui suit).

La figure 23 donne le schéma de synthèse spécifique d'une benzoxazine borée conforme à l'invention, de formule (A-7), par borylation de la benzoxazine bromée de formule (Ao-7) précédente à l'aide d'un ester diboronique (bis-pinacol ester de l'acide borique) (composé 4), cette benzoxazine borée selon l'invention étant utilisable in fine comme monomère de départ (Monomère noté M-7) pour la synthèse d'une polybenzoxazine (polymère noté P-5 à la figure 24 qui suit). On note que la formule (A-7) de cet exemple correspond à la formule (A-1-a) décrite précédemment.

Finalement, la figure 24 décrit le mode de synthèse spécifique d'un exemple de polymère polybenzoxazine particulier (Polymère P-5) à partir de la benzoxazine borée spécifique précédente selon l'invention de formule (A-7) (Monomère M-7) et de sa benzoxazine équivalente de départ de formule (Ao-7) (Monomère Mo-7) du type bromée, ainsi que cette même polybenzoxazine (Polymère noté P-5') une fois ses cycles oxazine ouverts après traitement thermique du Polymère P-5.

Typiquement, la polybenzoxazine issue du composé benzoxazine de l'invention peut comporter de dix à plusieurs centaines, préférentiellement de 50 à 300 unités structurelles à motifs de formule (I) et/ou (II), en particulier d'unités structurelles telles que représentées à titre d'exemples aux figures 14 à 18, 21 et 24.

Cette polybenzoxazine issue de la benzoxazine de l'invention est avantageusement utilisable, à titre de primaire d'adhésion ou comme couche adhésive unique, pour revêtir un substrat en métal, tout au moins un substrat dont au moins la surface est au moins en partie métallique, et faire adhérer ce dernier à du caoutchouc. Elle est tout particulièrement utilisable sur tout type de renfort métallique, tel que par exemple un fil, un film ou un câble en acier, notamment en acier au carbone, destiné en particulier à renforcer une matrice de caoutchouc insaturé tel que du caoutchouc naturel. Pour faire adhérer le caoutchouc à la couche de polybenzoxazine, on pourra aussi utiliser tout système adhésif connu, par exemple une colle textile conventionnelle du type "RFL" (résorcinol-formaldéhyde-latex). L'homme du métier comprendra aisément que la connexion entre le substrat métallique pourvu de sa couche de polybenzoxazine et la couche de caoutchouc avec laquelle il est au contact, sera assurée définitivement lors de la cuisson (réticulation) finale de l'article en caoutchouc concerné.

### 5. EXEMPLES DE REALISATION DE L'INVENTION

Dans la présente demande, sauf indication expresse différente, tous les pourcentages (%) indiqués sont des % en masse.

Les essais qui suivent décrivent :
- tout d'abord la fabrication d'une benzoxazine bromée de départ (Monomère Mo-7) de formule (Ao-7) : 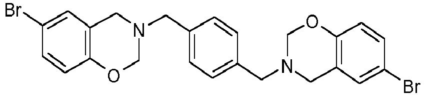
- puis la synthèse, à partir de cette dernière, de la benzoxazine borée conforme à l'invention (Monomère M-7), de formule (A-7) : 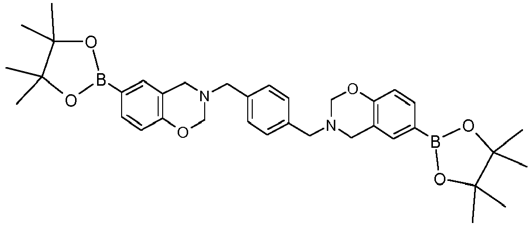
- puis la synthèse selon un procédé conforme à l'invention, par polycondensation des monomères Mo-7 et M-7, d'une polybenzoxazine (Polymère P-5) de formule (I-5) : 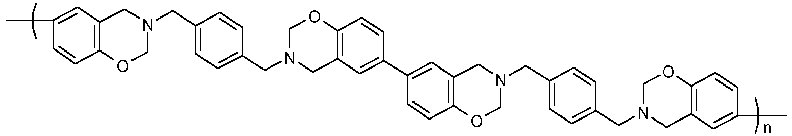
- enfin, des tests d'adhésion sont conduits pour illustrer l'excellente performance adhésive de cette polybenzoxazine (polymère P-5) issue du composé (Monomère M-7) de l'invention.

### 5.1. Synthèse de la benzoxazine bromée de départ (Monomère Mo-7)

On dispose pour cette synthèse d'un ballon rond à trois cols de 250 ml, équipé d'un thermomètre, d'une entrée d'azote, d'un agitateur magnétique et d'un réfrigérant.

La synthèse est réalisée selon le mode opératoire schématisé à la figure 22, comme expliqué en détail ci-après, à partir de trois composés : phénol halogéné (composé 1 ; 4-bromophénol ; produit Aldrich B75808), une diamine aromatique (composé 5 ; p-xylylène diamine ; produit TCI Europe D1018) et paraformaldéhyde (composé 3 ; produit Aldrich 158127), en présence de deux solvants (toluène et éthanol anhydres).

On verse dans le ballon le composé 1 (2 eq ; 10,38 g, soit 60 mmol) puis de l'éthanol (51 ml). La présence d'éthanol est ici importante, empêchant la formation de produit intermédiaire du type triazine, instable. Sous agitation, on introduit ensuite le composé 5 (1 eq ; 4,13 g soit 30 mmol), le composé 3 (4 eq ; 3,60 g soit 120 mmol) et enfin le toluène (102 ml). Le milieu réactionnel est chauffé (environ 75°C) à reflux pendant 51 h, puis les solvants et résidus volatils sont distillés à 110°C (sous vide de 1 mbar) pour évaporation. Le produit final est ensuite lavé (100 ml méthanol) et séché ; on obtient ainsi une poudre de couleur jaune.

Cette poudre est placée dans du méthanol (100 ml pour 15 g de poudre) et on chauffe à reflux (65°C) pendant 30 min. Puis on laisse la solution refroidir à température ambiante (environ 20°C) pour cristallisation du monomère. Le produit solide obtenu est isolé par filtration (filtre Büchner). On obtient ainsi une poudre de couleur blanche, après un séchage à l'étuve sous vide à 50°C, durant toute une nuit (rendement de réaction égal à environ 82%).

Le spectre RMN ¹H (500 MHz) du Monomère Mo-7 ainsi synthétisé, dissous dans du chloroforme, a confirmé sa structure chimique, avec les résultats suivants:
*3.89 (s, 4H), 3.94 (s, 4H), 4.87 (s, 4H), 6.70 (s, 1H), 6.72 (s, 1H), 7.06 (s, 2H), 7.23 (s, 2H), 7.32 (s, 4H).*

### 5.2. Synthèse de la benzoxazine borée conforme à l'invention (Monomère M-7)

La synthèse est réalisée selon le mode opératoire schématisé à la figure 23, comme expliqué en détail ci-après, à partir de deux composés : le monomère de départ Mo-7 (préalablement séché sous vide à 60°C toute la nuit) et un hétérocycle diboronique (composé 4), disponible commercialement (CAS 73183-34-3 ; produit Sigma Aldrich N° 697230 ; pureté 99%) qui est le bis-pinacol ester de l'acide diboronique, de formule :

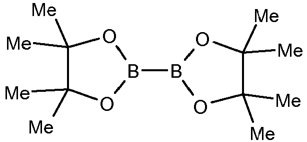

Cet hétérocycle répond bien à la formule (a) citée supra dans laquelle R₁, R₂, R₃ et R₄ représentent chacun un groupe méthyle (« Me »).

La synthèse est conduite dans un ballon rond à trois cols de 25 ml, équipé d'un bouchon à jupe rabattable, d'une entrée d'azote, d'un thermomètre, d'un agitateur magnétique et d'un réfrigérant, le tout en présence de solvant DMF anhydre (*N,N-*diméthylformamide ; produit Acros Organics N° 326871000) et d'acétate de potassium anhydre (produit Sigma Aldrich N° 791733) séché préalablement sous vide pendant 12 h à une température de 100°C.

L'appareillage est séché sous vide en utilisant un pistolet à air chaud jusqu'à ce que le thermomètre atteigne une température d'au moins 100°C dans le ballon de réaction. On laisse l'ensemble refroidir à température ambiante (20°C), puis l'appareillage est mis sous courant d'azote pendant toute la synthèse.

Sous agitation, on introduit alors successivement dans le ballon 200 mg de monomère Mo-7, 6 ml de DMF, 111 mg d'acétate de potassium et 211 mg de Composé 4. Puis on injecte à l'aide d'une seringue une solution, dans 2 ml de DMF, de 16,5 mg de catalyseur Pd(dppf)Cl₂ (produit Sigma Aldrich N° 697230) pesé dans une boîte à gants sous courant d'azote.

Le mélange réactionnel est chauffé à 80°C pendant 40 h, transvasé dans un ballon de 50 ml puis le solvant DMF distillé à l'aide d'un évaporateur rotatif à 95°C sous 35 mbar. On laisse refroidir le résidu de distillation à température ambiante (20°C), puis sous agitation pendant 10 min avec 16 ml d'eau déionisée.

La suspension ainsi obtenue est centrifugée à 10 000 tours/min durant 5 min. Le produit obtenu est ensuite transvasé dans un récipient contenant 40 ml de chloroforme et 10 ml d'eau déionisée, le tout agité à température ambiante pendant 10 min. La phase aqueuse est extraite deux fois avec 40 ml de chloroforme, puis la phase organique est séchée sur sulfate de sodium, filtrée, enfin le chloroforme évaporé à l'aide d'un évaporateur rotatif. Le produit ainsi obtenu est purifié par chromatographie sur gel de silice, puis élué avec un mélange d'acétate d'éthyle/cyclohexane (1:3).

Le spectre RMN ¹H (500 MHz) du produit final (Monomère M-7) ainsi synthétisé, dissous dans du CD₂Cl₂, a confirmé sa structure chimique, avec les résultats suivants:
*1.31 (s, 24H), 3.89 (s, 4H), 3.97 (s, 4H), 3.89 (s, 4H), 3.97 (s, 4H), 4.90 (s, 4H), 6.77 (d, 2H), 7.31 (m, 4H), 7.37 (m, 2H), 7.51 (d, 1H), 7.53 (d, 1H).*

### 5.3. Synthèse de la polybenzoxazine (Polymère P-5)

Cette synthèse est réalisée selon le mode opératoire schématisé à la figure 24, comme décrit en détail ci-après, à partir de deux monomères : la benzoxazine de l'invention obtenue à l'étape précédente (Monomère M-7) et sa benzoxazine bromée de départ (Monomère Mo-7), le tout en présence de carbonate de potassium (K₂CO₃ ; produit Riedel-de Haën N°31245), de solvant (anhydre) DMF (*N,N-*diméthylformamide ; produit Acros Organics N°326871000). Les deux monomères (Mo-7 et M-7) sont préalablement séchés sous vide à 50°C toute la nuit, de même pour le carbonate de potassium mais à une température de 150°C.

La synthèse est conduite dans un ballon rond à trois cols de 50 ml, équipé d'un bouchon à jupe rabattable, d'une entrée d'azote, d'un thermomètre, d'un agitateur magnétique et un réfrigérant. L'appareillage est séché sous vide en utilisant un pistolet à air chaud jusqu'à ce que le thermomètre atteigne une température d'au moins 100°C dans le ballon de réaction. On laisse l'ensemble refroidir à température ambiante (20°C), puis l'appareillage est mis sous courant d'azote pendant toute la synthèse.

Sous agitation, on introduit successivement dans le ballon, le Monomère M-7 (1 eq ; 0,106 g soit 0,2 mmol) de formule (A-7), puis le Monomère Mo-7 de formule (Ao-7) (1 eq ; 0,125 g soit 0,2 mmol). On ajoute ensuite 10 ml de DMF (solvant des deux monomères), puis à titre de base K₂CO₃ (5 eq ; 0,149 g soit 1 mmol). Puis on injecte à l'aide d'une seringue une solution, dans 4 ml de DMF, de 2,9 mg de catalyseur Pd(dppf)Cl₂ pesé dans une boîte à gants sous courant d'azote.

Le mélange réactionnel est chauffé à 80°C pendant 48 h, puis on laisse refroidir à température ambiante ; le mélange est finalement versé dans 50 ml d'eau déionisée, le tout agité fortement (barreau magnétique) pendant 30 min à 20°C ; au cours de ce lavage, pour extraire le carbonate de potassium, on ajoute de l'acide (HCl 1%) goutte à goutte jusqu'à pH neutre. Le précipité ainsi obtenu est isolé par filtration (filtre Büchner) et lavé (deux fois) avec 50 ml d'eau distillée, puis séché sous vide à 40°C pendant toute une nuit (environ 12 h).

Le Polymère P-5 de la Fig. 24 a été ainsi obtenu, comme attesté par l'analyse RMN 1H (500 MHz) dans le solvant DMA-*d9*, qui a donné les résultats suivants :
3. *95 (s, 4H), 4.03 (s, 4H), 4.94 (s, 4H), 6.80-6.82 (d, 2H), 6.87-6.89 (d, 2H), 7.28-7.29 (m, 4H), 7.36-7.37 (m, 3H), 7.43-7.46 (m, 2H).*

Ce Polymère P-5, sous forme de poudre de couleur beige, a été également analysé par DSC (*Differential Scanning Calorimetry*) entre -80°C et +320°C selon une rampe de 10°C/min (appareil DSC "822-2" de Mettler Toledo ; atmosphère azote). L'analyse a montré au premier passage (entre -80°C et +320°C) une exothermie (correspondant à l'ouverture des cycles oxazine, et à la réticulation du polymère) au-delà de 200°C, avec un maximum à environ 260°C. Au cours des deuxième et troisième passages de DSC conduits entre -80°C et +320°C, aucune transition vitreuse apparente (Tg) n'a été visible.

### 5.4. Test d'adhésion dans un composite métal / caoutchouc

Une partie (325 mg) du Polymère P-5 précédemment préparé a été dissoute dans 8 ml de DMPU (1,3-diméthyl-3,4,5,6-tetrahydro-2(1H)-pyrimidinone ; produit Sigma Aldrich 41661) avec 10% en poids d'accélérateur « DY 9577 ES » (produit Huntsman), ceci pour former une solution dont une fraction (0,6 ml) a été ensuite déposée de manière uniforme sur un ruban (film) en laiton de dimensions 10 cm x 2,5 cm et d'épaisseur 0,5 mm ; l'ensemble a été placé à l'étuve à 175°C (ventilation à l'air) durant 5 min, puis 5 min supplémentaires à 230°C sous vide afin d'une part d'éliminer toute trace de solvant et d'autre part d'ouvrir au moins en partie (c'est-à-dire totalement ou partiellement) les cycles oxazine du polymère, cette dernière étape s'accompagnant d'un changement prononcé de couleur du polymère, passant au orange foncé.

Après refroidissement à température ambiante, le ruban pourvu en surface de sa fine couche (épaisseur 5 à 10 µm) de polybenzoxazine ainsi formée, a été ensuite soumis à une opération d'encollage conventionnelle en deux temps (encollage deux bains), tout d'abord par immersion dans un premier bain aqueux (environ 94% d'eau) à base de résine époxy (polyglycérol polyglycidyl éther, environ 1%) et de composé isocyanate (bloqué caprolactame, environ 5%), première étape d'encollage suivie d'un séchage (2 min à 100°C) puis d'un traitement thermique (5 min à 200°C). Puis le ruban ainsi traité a été immergé dans un second bain aqueux de colle RFL (environ 81% en poids d'eau) à base de résorcinol (environ 2%), formol (environ 1%) et d'un latex de caoutchouc (environ 16% de caoutchoucs NR, SBR et VP-SBR) ; il a été enfin séché au four pendant 2 min à 130°C, puis traité thermiquement 5 min à 200°C.

Le ruban en laiton ainsi revêtu du film de polybenzoxazine puis encollé, a été ensuite placé entre deux couches de composition de caoutchouc conventionnelle pour armature de ceinture de bandage pour véhicule tourisme, composition à base de caoutchouc naturel, de noir de carbone et silice à titre de charge, et d'un système de vulcanisation (soufre et accélérateur sulfénamide) ; cette composition étant dépourvue de sel de cobalt. Puis l'éprouvette de composite métal / caoutchouc ainsi préparée a été placée sous presse et le tout cuit (vulcanisé) à 150°C pendant 30 min sous une pression de 20 bar.

Après vulcanisation du caoutchouc, on a obtenu un excellent collage entre la matrice de caoutchouc et le ruban métallique malgré l'absence de sel de cobalt dans la matrice de caoutchouc : lors de tests de pelage (à 20°C), on a constaté en effet que la rupture se produisait systématiquement dans la matrice de caoutchouc elle-même et non à l'interphase entre métal et caoutchouc. D'autres essais de collage ont été conduits sur un ruban en acier clair (non revêtu) ; ils ont révélé eux aussi une excellente adhésion au caoutchouc (rupture systématique dans la matrice de caoutchouc).

En conclusion, la benzoxazine borée selon l'invention permet la synthèse de polymères offrant aux renforts métalliques l'avantage majeur de pouvoir ensuite être collés à des matrices de caoutchouc en utilisant de simples colles textiles telles que des colles RFL, ou encore directement (c'est-à-dire sans emploi de telles colles) à ces matrices de caoutchouc, par exemple lorsque ces dernières contiennent des élastomères insaturés fonctionnalisés appropriés tels que des élastomères époxydés.

Ainsi, peuvent être utilisés des renforts métalliques revêtus ou non de couches métalliques adhésives telles que du laiton, ainsi que des matrices de caoutchouc environnantes dépourvues de sels métalliques, en particulier de sels de cobalt.

En outre, ceci constituant un avantage notable comparativement aux autres polymères connus décrits en introduction du présent mémoire, les polybenzoxazines issues des benzoxazines borées de l'invention ont la capacité remarquable, à haute température, d'ouvrir leurs cycles oxazine et de conduire ainsi à une structure de résine polyphénolique thermodurcissable. Ceci leur confère une meilleure stabilité thermique. Leur microstructure spécifique permet enfin, très avantageusement, d'ajuster la flexibilité de la molécule selon les applications particulières visées.

## Revendications

1. Composé benzoxazine borée répondant à la formule : 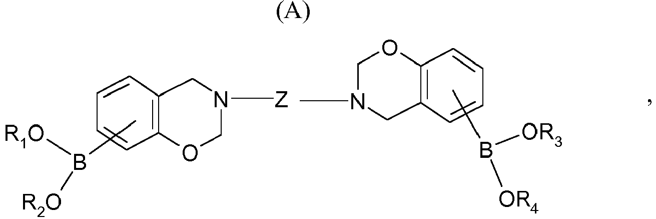 dans laquelle :
- Z représente un groupement de liaison au moins divalent, aliphatique, cycloaliphatique ou aromatique, comportant au moins un atome de carbone et optionnellement au moins un hétéroatome choisi parmi O, S et P ;
- R₁, R₂, R₃ et R₄, identiques ou différents, représentent l'hydrogène ou un alkyle comportant 1 à 12 atomes de carbone, R₁ et R₂ d'une part, R₃ et R₄ d'autre part pouvant optionnellement former un hétérocycle avec les deux atomes d'oxygène et l'atome de bore auxquels ils sont respectivement liés.

2. Composé selon la revendication 1, dans lequel Z comporte un groupement aromatique comportant 6 à 30, de préférence 6 à 20 atomes de carbone.

3. Composé selon la revendication 2, répondant à la formule (A-1) :

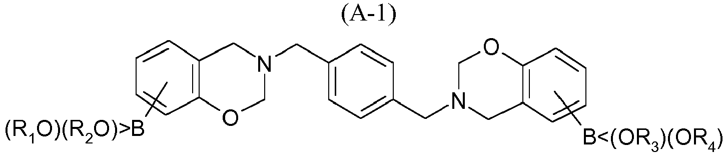

4. Composé selon la revendication 1, dans lequel Z représente un groupement aliphatique comportant 1 à 20, de préférence 1 à 16 atomes de carbone, ou un groupement cycloaliphatique comportant 3 à 20, de préférence 3 à 16 atomes de carbone, et optionnellement au moins un hétéroatome choisi parmi O et S.

5. Composé selon la revendication 4, dans lequel Z représente un enchaînement (poly)alkylène comportant 1 à 20, de préférence 1 à 16 atomes de carbone, et optionnellement au moins un hétéroatome choisi parmi O et S.

6. Composé selon la revendication 5, répondant à l'une des formules (A-2) à (A-5) :

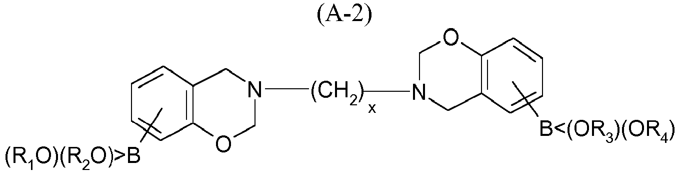

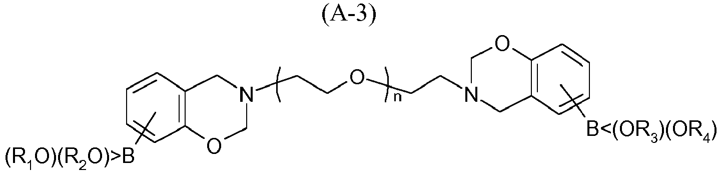

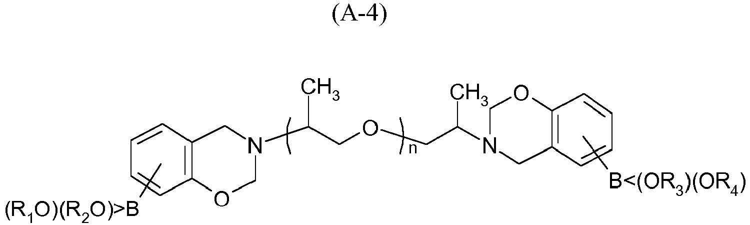

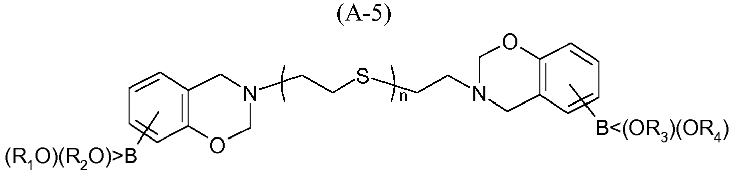

7. Composé selon l'une quelconque des revendications 1 à 6, R₁ et R₂ d'une part, R₃ et R₄ d'autre part formant un hétérocycle avec les deux atomes d'oxygène et l'atome de bore auxquels ils sont respectivement liés.

8. Composé selon la revendication 7, chaque hétérocycle répondant à l'une des formules (a), (b) ou (c) ci-dessous :

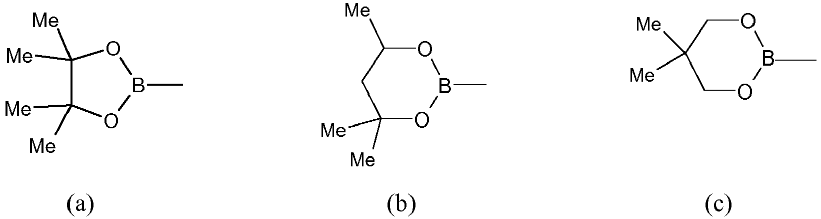

9. Composé selon l'une quelconque des revendications 1 à 8, l'atome de bore porté par chaque noyau benzénique des deux cycles oxazine étant situé en position para de l'oxygène du cycle oxazine.

10. Composé selon les revendications 8 et 9, répondant à l'une des formules (A-1-a) à (A-5-a) ci-dessous : 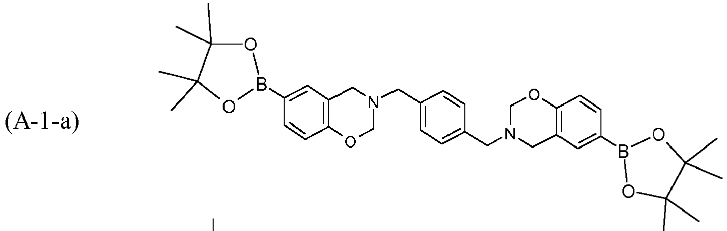 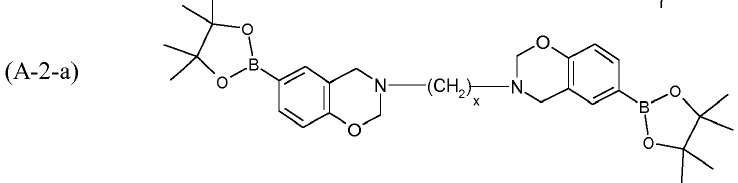 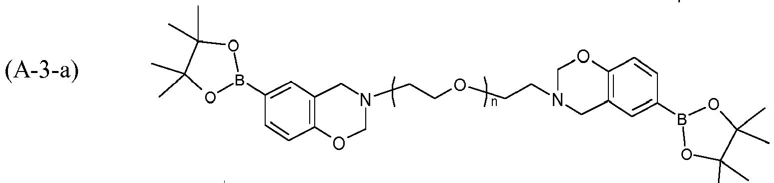 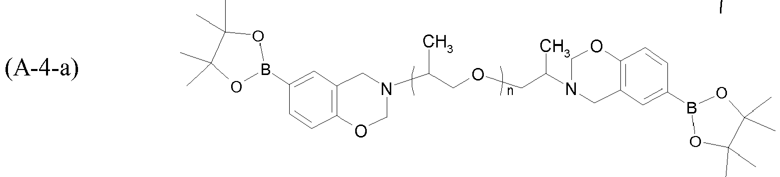 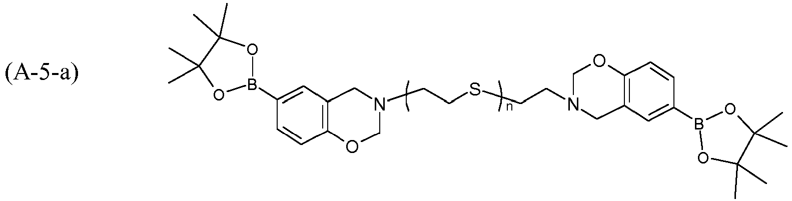 dans lesquelles les symboles « x » et « n » représentent un nombre entier, de préférence un nombre entier tel que Z comporte 1 à 20 atomes de carbone.

11. Utilisation d'un composé selon l'une quelconque des revendications 1 à 10, pour la synthèse d'une polybenzoxazine.

12. Procédé de synthèse d'une polybenzoxazine, par polycondensation d'une benzoxazine selon l'une quelconque des revendications 1 à 10.

13. Procédé selon la revendication 12, par polycondensation, à titre de premier monomère, d'une benzoxazine selon l'une quelconque des revendications 1 à 10, avec au moins, à titre de deuxième monomère, une benzoxazine bromée ayant pour formule (B) : 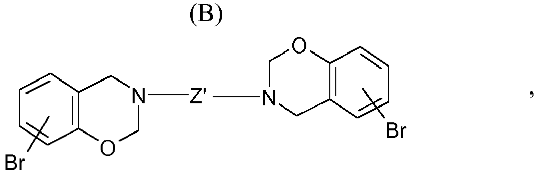 dans laquelle Z', identique à ou différent de Z, représente un groupement de liaison au moins divalent, aliphatique, cycloaliphatique ou aromatique, comportant au moins un atome de carbone et optionnellement au moins un hétéroatome choisi parmi O, S et P.

14. Procédé selon la revendication 13, dans lequel Z' comporte un groupement aromatique comportant 6 à 30, de préférence 6 à 20 atomes de carbone.

15. Procédé selon la revendication 13, dans lequel Z' représente un enchaînement (poly)alkylène comportant 1 à 20, de préférence 1 à 16 atomes de carbone.

## Patentansprüche

1. Borierte Benzoxazinverbindung der Formel: 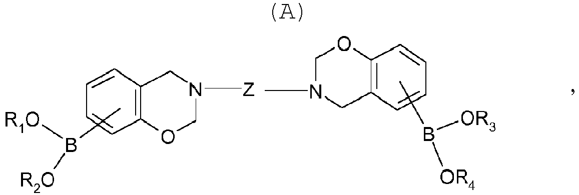 in der:
- Z für eine mindestens zweiwertige aliphatische, cycloaliphatische oder aromatische Verknüpfungsgruppe, die mindestens ein Kohlenstoffatom und gegebenenfalls mindestens ein aus O, S und P ausgewähltes Heteroatom umfasst, steht;
- R₁, R₂, R₃ und R₄ gleich oder verschieden sind und für Wasserstoff oder ein Alkyl mit 1 bis 12 Kohlenstoffatomen stehen, wobei R₁ und R₂ einerseits und R₃ und R₄ andererseits gegebenenfalls mit den beiden Sauerstoffatomen und dem Boratom, an das sie jeweils gebunden sind, einen Heterocyclus bilden können.

2. Verbindung nach Anspruch 1, wobei Z eine aromatische Gruppe mit 6 bis 30 und vorzugsweise 6 bis 20 Kohlenstoffatomen umfasst.

3. Verbindung nach Anspruch 2, die der Formel (A-1) entspricht:

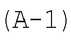

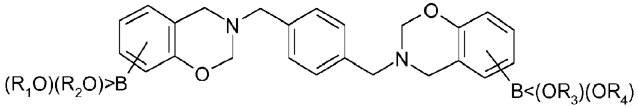

4. Verbindung nach Anspruch 1, wobei Z für eine aliphatische Gruppe mit 1 bis 20 und vorzugsweise 1 bis 16 Kohlenstoffatomen oder eine cycloaliphatische Gruppe mit 3 bis 20 und vorzugsweise 3 bis 16 Kohlenstoffatomen und gegebenenfalls mindestens einem aus O und S ausgewählten Heteroatom steht.

5. Verbindung nach Anspruch 4, wobei Z für eine (Poly)alkylenkette mit 1 bis 20 und vorzugsweise 1 bis 16 Kohlenstoffatomen und gegebenenfalls mindestens einem aus O und S ausgewählten Heteroatom steht.

6. Verbindung nach Anspruch 5, die einer der Formeln (A-2) bis (A-5) entspricht:

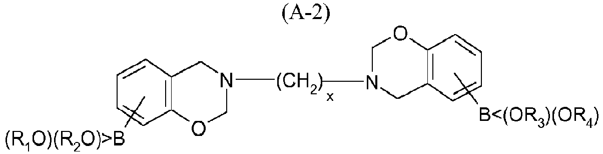

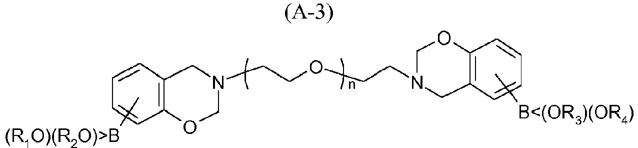

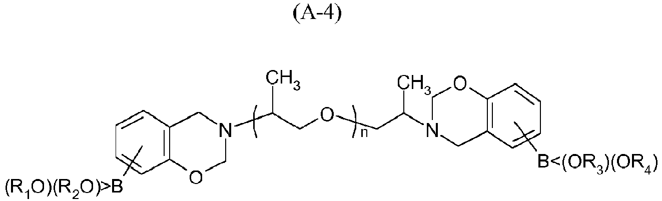

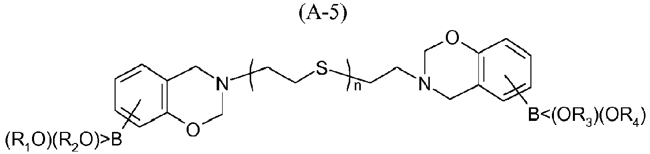

7. Verbindung nach einem der Ansprüche 1 bis 6, wobei R₁ und R₂ einerseits und R₃ und R₄ andererseits mit den beiden Sauerstoffatomen und dem Boratom, an das sie jeweils gebunden sind, einen Heterocyclus bilden.

8. Verbindung nach Anspruch 7, wobei jeder Heterocyclus einer der nachstehenden Formeln (a), (b) oder (c) entspricht:

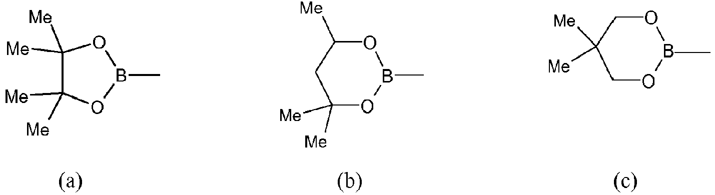

9. Verbindung nach einem der Ansprüche 1 bis 8, wobei das Boratom an jedem Benzolkern der beiden Oxazinringe in para-Position zum Sauerstoff des Oxazinrings steht.

10. Verbindung nach den Ansprüchen 8 und 9, die einer der nachstehenden Formeln (A-1-a) bis (A-5-a) entspricht: 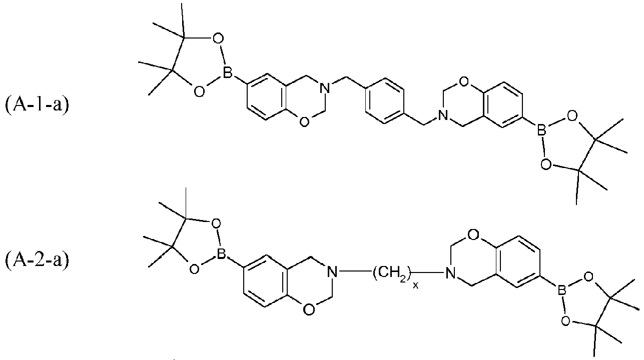 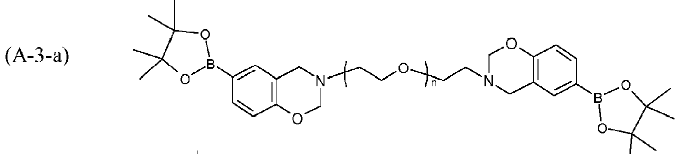 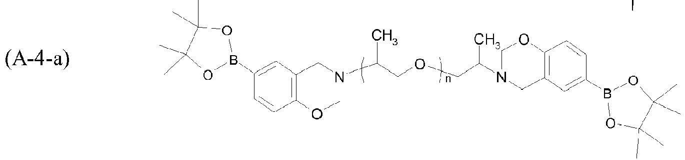 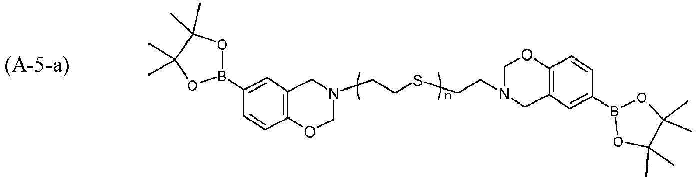 in denen die Symbole "x" und "n" für eine ganze Zahl stehen, vorzugsweise für eine solche ganze Zahl, dass Z 1 bis 20 Kohlenstoffatome umfasst.

11. Verwendung einer Verbindung nach einem der Ansprüche 1 bis 10 zur Synthese eines Polybenzoxazins.

12. Verfahren zur Synthese eines Polybenzoxazins durch Polykondensation eines Benzoxazins nach einem der Ansprüche 1 bis 10.

13. Verfahren nach Anspruch 12 durch Polykondensation eines Benzoxazins nach einem der Ansprüche 1 bis 10 als erstes Monomer mit mindestens einem bromierten Benzoxazin der Formel (B): 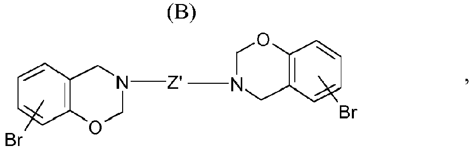 in der Z' mit Z identisch oder davon verschieden ist und für eine mindestens zweiwertige aliphatische, cycloaliphatische oder aromatische Verknüpfungsgruppe, die mindestens ein Kohlenstoffatom und gegebenenfalls mindestens ein aus O, S und P ausgewähltes Heteroatom umfasst, steht, als zweitem Monomer.

14. Verfahren nach Anspruch 13, wobei Z' eine aromatische Gruppe mit 6 bis 30 und vorzugsweise 6 bis 20 Kohlenstoffatomen umfasst.

15. Verfahren nach Anspruch 13, wobei Z' für eine (Poly)alkylenkette mit 1 bis 20 und vorzugsweise 1 bis 16 Kohlenstoffatomen steht.

## Claims

1. Borated benzoxazine compound corresponding to the formula: 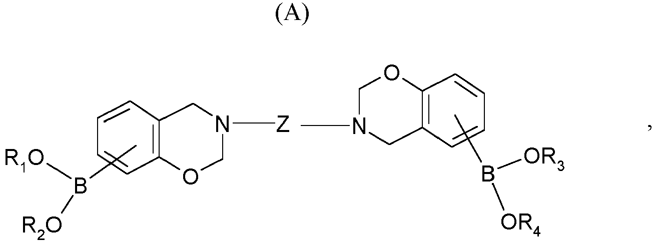 in which:
- Z represents an at least divalent, aliphatic, cycloaliphatic or aromatic, bonding group comprising at least one carbon atom and optionally at least one heteroatom chosen from O, S and P;
- R₁, R₂, R₃ and R₄, which are identical or different, represent hydrogen or an alkyl comprising from 1 to 12 carbon atoms, it being possible for R₁ and R₂, on the one hand, R₃ and R₄, on the other hand, optionally to form a heterocycle with the two oxygen atoms and the boron atom to which they are respectively bonded.

2. Compound according to Claim 1, in which Z comprises an aromatic group comprising from 6 to 30, preferably from 6 to 20, carbon atoms.

3. Compound according to Claim 2, corresponding to the formula (A-1):

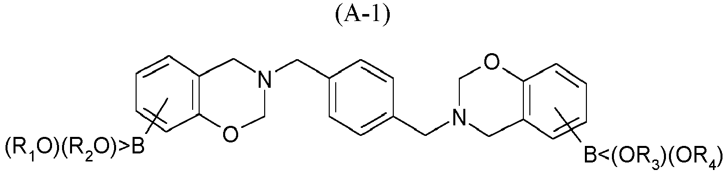

4. Compound according to Claim 1, in which Z represents an aliphatic group comprising from 1 to 20, preferably from 1 to 16, carbon atoms, or a cycloaliphatic group comprising from 3 to 20, preferably from 3 to 16, carbon atoms, and optionally at least one heteroatom chosen from O and S.

5. Compound according to Claim 4, in which Z represents a (poly)alkylene sequence comprising from 1 to 20, preferably from 1 to 16, carbon atoms and optionally at least one heteroatom chosen from O and S.

6. Compound according to Claim 5, corresponding to one of the formulae (A-2) to (A-5):

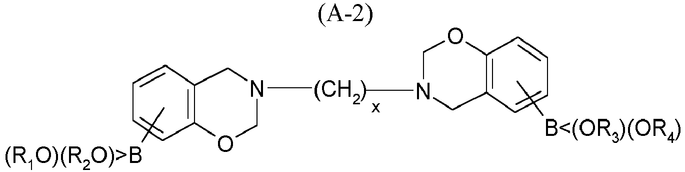

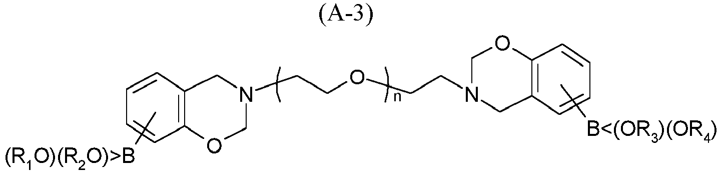

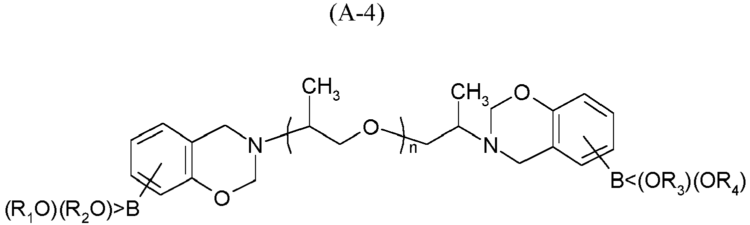

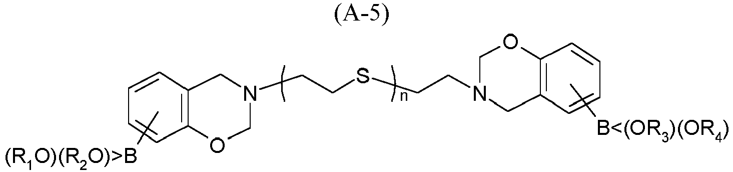

7. Compound according to any one of Claims 1 to 6, R₁ and R₂, on the one hand, R₃ and R₄, on the other hand, forming a heterocycle with the two oxygen atoms and the boron atom to which they are respectively bonded.

8. Compound according to Claim 7, each heterocycle corresponding to one of the formulae (a), (b) or (c) below:

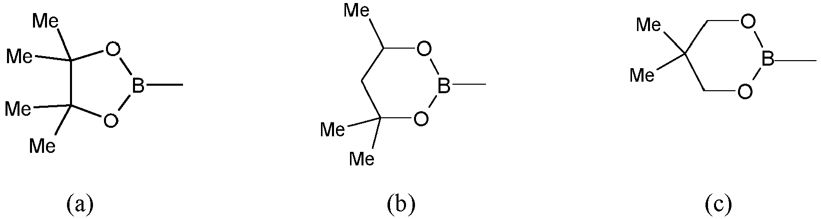

9. Compound according to any one of Claims 1 to 8, the boron atom borne by each benzene nucleus of the two oxazine rings being located in the para position with respect to the oxygen of the oxazine ring.

10. Compound according to Claims 8 and 9, corresponding to one of the formulae (A-1-a) to (A-5-a) below: 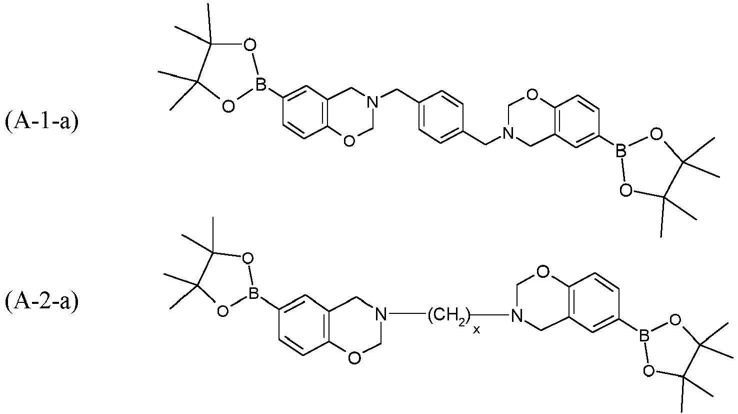 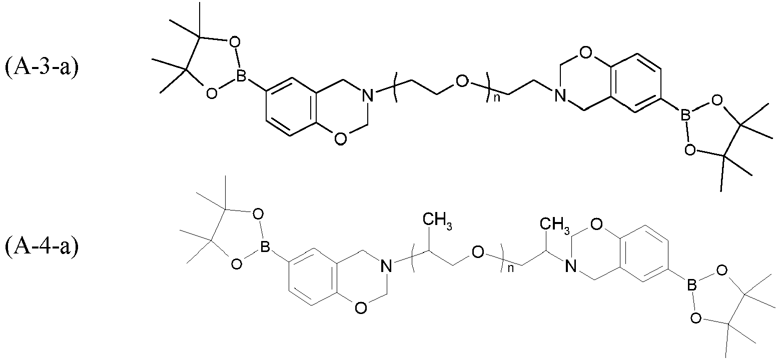 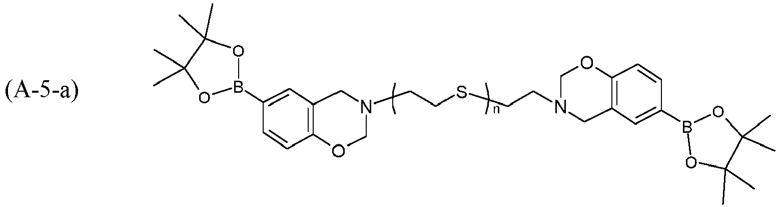 in which the symbols "x" and "n" represent an integer, preferably an integer such that Z comprises from 1 to 20 carbon atoms.

11. Use of a compound according to any one of Claims 1 to 10 in the synthesis of a polybenzoxazine.

12. Process for the synthesis of a polybenzoxazine by polycondensation of a benzoxazine according to any one of Claims 1 to 10.

13. Process according to Claim 15, by polycondensation, as first monomer, of a benzoxazine according to any one of Claims 1 to 10 with at least, as second monomer, a brominated benzoxazine having the formula (B): 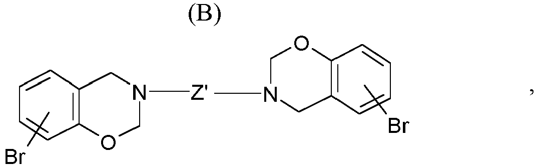 in which Z', which is identical to or different from Z, represents an at least divalent, aliphatic, cycloaliphatic or aromatic, bonding group comprising at least one carbon atom and optionally at least one heteroatom chosen from O, S and P.

14. Process according to Claim 13, in which Z' comprises an aromatic group comprising from 6 to 30, preferably from 6 to 20, carbon atoms.

15. Process according to Claim 13, in which Z' represents a (poly)alkylene sequence comprising from 1 to 20, preferably from 1 to 16, carbon atoms.
